(19) Europäisches Patentamt
European Patent Office
Office européen des brevets

(11) **EP 4 548 871 A2**

(12) **EUROPEAN PATENT APPLICATION**

(43) Date of publication:
**07.05.2025  Bulletin 2025/19**

(21) Application number: **25164718.6**

(22) Date of filing: **17.08.2018**

(51) International Patent Classification (IPC):
***A61B 18/14*** (2006.01)

(52) Cooperative Patent Classification (CPC):
**A61B 18/1492;** A61B 2018/00077;
A61B 2018/00083; A61B 2018/00107;
A61B 2018/0016; A61B 2018/0022;
A61B 2018/00267; A61B 2018/00517;
A61B 2018/00577; A61B 2018/00797;
A61B 2018/00821

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2017  US 201762546911 P**

(62) Document number(s) of the earlier application(s) in
accordance with Art. 76 EPC:
**22201230.4 / 4 137 085**
**18769900.4 / 3 624 718**

(71) Applicant: **St. Jude Medical, Cardiology Division,
Inc.
St. Paul, MN 55117 (US)**

(72) Inventors:
• **OLSON, Gregory K.
Elk River, 55330 (US)**
• **PARSONAGE, Ed
St. Paul, 55116 (US)**

• **TEGG, Troy T.
Elk River, 55330 (US)**
• **EBNER, Bruce
Shorewood, 55331 (US)**
• **DAHLEN, Travis
Forest Lake, 55025 (US)**
• **MANDA, Rishi
Stillwater, 55082 (US)**

(74) Representative: **Mathys & Squire
The Shard
32 London Bridge Street
London SE1 9SG (GB)**

Remarks:
This application was filed on 19.03.2025 as a
divisional application to the application mentioned
under INID code 62.

(54) **TEMPERATURE SENSOR AND THREE-DIMENSIONAL ELECTRODE**

(57)    A medical device includes an elongate shaft extending along a shaft longitudinal axis and includes a shaft proximal portion and a shaft distal portion. The medical device can include an electrode disposed on the shaft distal portion. The medical device can include a first conductor lead and a second conductor lead, each of the conductor leads electrically being coupled to the electrode. A thermocouple junction formed via a thermocouple conductor can be electrically coupled to the electrode and the first conductor lead.

FIG. 1

EP 4 548 871 A2

## Description

BACKGROUND

a. Field

The instant disclosure relates to a temperature sensor and three-dimensional electrode.

b. Background Art

Medical devices, catheters, and/or cardiovascular catheters, such as electrophysiology catheters can be used in a variety of diagnostic, therapeutic, mapping and/or ablative procedures to diagnose and/or correct conditions such as atrial arrhythmias, including for example, ectopic atrial tachycardia, atrial fibrillation, and/or atrial flutter. Arrhythmias can create a variety of conditions including irregular heart rates, loss of synchronous atrioventricular contractions and/or stasis of blood flow in a chamber of a heart, which can lead to a variety of symptomatic and asymptomatic ailments and even death.

A medical device can be threaded through a vasculature of a patient to a site where the diagnostic, therapeutic, mapping, and/or ablative procedure to diagnose and/or correct the condition is performed. To aid in the delivery of the medical device to the site, sensors (e.g., electrodes) can be placed on the medical device, which can receive signals that are generated proximate to the patient from a device (e.g., electromagnetic field generator). Based on the received signals, an orientation and/or position of the medical device can be computed.

Once the medical device has reached the site, one or more of the variety of diagnostic, therapeutic, mapping and/or ablative procedures to diagnose and/or correct conditions can be performed. In some of those procedures, it can be beneficial to attain a temperature at the site. Accordingly, a temperature sensor can be included on the medical device and can be configured to measure a temperature at the site.

SUMMARY

Various embodiments of the present disclosure include a medical device that includes an elongate shaft extending along a shaft longitudinal axis and includes a shaft proximal portion and a shaft distal portion. The medical device can include an electrode disposed on the shaft distal portion. The medical device can include a first conductor lead and a second conductor lead, each of the conductor leads electrically being coupled to the electrode. A thermocouple junction formed via a thermocouple conductor can be electrically coupled to the electrode and the first conductor lead.

Various embodiments of the present disclosure include a medical device that includes an elongate shaft extending along a shaft longitudinal axis and includes a shaft proximal portion and a shaft distal portion. An electrode can be disposed on the shaft distal portion, the electrode including a three-dimensional profile portion, the three-dimensional profile portion extending upwardly from a surface. A face can extend across a top of the upwardly extending profile portion, wherein the electrode is disposed on the face.

Various embodiments of the present disclosure can include a medical device. The medical device can include an elongate shaft extending along a shaft longitudinal axis and comprising a shaft proximal portion and a shaft distal portion. An ablation tip can be connected to the shaft distal portion, wherein the ablation tip is a conductive shell disposed over an end of the shaft distal portion. A printed temperature sensor can be thermally coupled with the conductive shell, wherein the temperature sensor is a printed temperature sensor and is configured to sense a temperature of the conductive shell. A pair of leads can extend along the elongate shaft and can be electrically coupled with the temperature sensor.

Various embodiments of the present disclosure include a medical device. The medical device can include an elongate shaft extending along a shaft longitudinal axis and comprising a shaft proximal portion and a shaft distal portion. A balloon can be disposed along the shaft distal. In some embodiments, a plurality of temperature sensors can be disposed on the balloon.

Various embodiments of the present disclosure can include a medical device. The medical device can include an elongate shaft extending along a shaft longitudinal axis and comprising a proximal portion and a distal portion. The medical device can include an elevated electrode disposed on the distal portion, wherein the elevated electrode is a spherical cap. The spherical cap includes a core formed from a first conductive material. An exterior of the core can be coated with a second conductive material.

BRIEF DESCRIPTION OF THE DRAWINGS

Fig. 1 is a diagrammatic view of an exemplary system for performing one or more diagnostic or therapeutic procedures, in accordance with embodiments of the present disclosure.
Fig. 2 is a diagrammatic top view of a temperature sensor, in accordance with embodiments of the present disclosure.
Fig. 3A is a diagrammatic top view of a three-dimensional electrode, in accordance with embodiments of the present disclosure.
Fig. 3B is a diagrammatic end view of the three-dimensional electrode depicted in Fig. 3A, in accordance with embodiments of the present disclosure.
Fig. 4A is an isometric view of a substrate blank for use in the formation of a three-dimensional electrode as further depicted in Figs. 4A and 4B, in accordance with embodiments of the present disclosure.

Fig. 4B is an isometric view of the substrate blank depicted in Fig. 4A with a three-dimensional profile portion formed in the substrate blank, in accordance with embodiments of the present disclosure.

Fig. 4C is an isometric cross-sectional view of the substrate blank depicted in Fig. 4B, in accordance with embodiments of the present disclosure.

Fig. 5A is an isometric view of a substrate blank for use in the formation of a three-dimensional electrode as further depicted in Fig. 5B, in accordance with embodiments of the present disclosure.

Fig. 5B is an isometric view of the substrate blank depicted in Fig. 5A with a three-dimensional profile portion formed in the substrate blank, in accordance with embodiments of the present disclosure.

Fig. 6 is a top view of a high density electrode mapping catheter, according to various embodiments of the present disclosure.

Fig. 7A is a side view of a renal denervation catheter, according to various embodiments of the present disclosure.

Fig. 7B is an end view of a renal denervation catheter, according to various embodiments of the present disclosure.

Fig. 8A is an isometric side view of a temperature enabled catheter tube with axially aligned temperature sensors, in accordance with embodiments of the present disclosure.

Fig. 8B is an isometric side view of a temperature enabled catheter tube with axially offset temperature sensors, in accordance with embodiments of the present disclosure.

Fig. 8C is an isometric side view of a second embodiment of a temperature enabled catheter tube with axially aligned temperature sensors, in accordance with embodiments of the present disclosure.

Fig. 8D is an isometric side view of a second embodiment of a temperature enabled catheter tube with axially offset temperature sensors, in accordance with embodiments of the present disclosure.

Fig. 9 is a diagrammatic isometric view of a temperature enabled catheter ablation tip, in accordance with embodiments of the present disclosure.

Fig. 10 is a side view of a temperature enabled ablation balloon, in accordance with embodiments of the present disclosure.

Fig. 11 is a diagrammatic isometric view of a temperature enabled catheter ablation tip that includes temperature sensors disposed on the ablation tip, in accordance with embodiments of the present disclosure.

Fig. 12 is a diagrammatic isometric view of a temperature enabled catheter tube with temperature sensors that share a common lead, in accordance with embodiments of the present disclosure.

Fig. 13 is a diagrammatic isometric view of a temperature enabled catheter tube with a temperature sensor in communication with a computing device, in accordance with embodiments of the present disclosure.

Fig. 14 is a top view of an array of temperature sensors that share a common lead, in accordance with embodiments of the present disclosure.

Fig. 15 is a side view of a radial ablation tip, according to various embodiments of the present disclosure.

Figs. 16A to 16J depict the construction of a temperature sensor and electrode, in accordance with embodiments of the present disclosure.

Figs. 17A to 17D depict various views of an elevated electrode, in accordance with embodiments of the present disclosure.

Fig. 18 depicts a graphical chart representing a difference in impedance of a traditional type electrode versus that of an elevated electrode, in accordance with embodiments of the present disclosure.

Aspects

1. A medical device, comprising:

an elongate shaft extending along a shaft longitudinal axis and comprising a shaft proximal portion and a shaft distal portion;
an electrode disposed on the shaft distal portion;
a first conductor lead and a second conductor lead, each of the conductor leads electrically coupled to the electrode;
a thermocouple junction formed via a thermocouple conductor electrically coupled to the electrode and the first conductor lead.

2. The medical device of aspect 1, wherein the first conductor lead, the second conductor lead, and the thermocouple conductor are disposed between the shaft distal portion and the electrode.

3. The medical device of aspect 1, wherein the first conductor lead, the second conductor lead, and the thermocouple conductor are disposed within the electrode.

4. The medical device of aspect 1, wherein the first conductor lead comprises a first conductor tip and the thermocouple conductor comprises a thermocouple tip.

5. The medical device of aspect 4, wherein the thermocouple junction comprises an electrical junction between the first conductor tip and the thermocouple tip.

6. The medical device of aspect 5, wherein:

the second conductor lead comprises a second conductor tip; and

the second conductor tip is disposed adjacent to the trace temperature sensor.

7. The medical device of aspect 1, wherein the electrode is disposed on a substrate that includes a three-dimensional profile portion.

8. The medical device of aspect 7, further comprising a plurality of electrodes, each of the electrodes including respective conductor leads and thermocouple conductors that form respective thermocouple junctions on each one of the plurality of electrodes, wherein the plurality of electrodes are formed in an array disposed on the shaft distal portion.

9. A medical device, comprising:

an elongate shaft extending along a shaft longitudinal axis and comprising a shaft proximal portion and a shaft distal portion; and
an electrode disposed on the shaft distal portion, wherein the electrode includes:

a three-dimensional profile portion, the three-dimensional profile portion extending upwardly from a surface; and
a face extending across a top of the upwardly extending profile portion, wherein the electrode is disposed on the face.

10. The medical device of aspect 9, wherein three-dimensional profile portion is formed via an additive process.

11. The medical device of aspect 9, wherein the three-dimensional profile portion is formed via a subtractive process.

12. The medical device of aspect 9, wherein the upwardly extending profile portion and the face define a profile space.

13. The medical device of aspect 12, wherein:

a conductive material is disposed on the face;
a first conductor lead, a second conductor lead, and a thermocouple conductor are disposed between the face and the conductive material, and
the first conductor lead, the second conductor lead, and the thermocouple conductor are electrically coupled with the conductive material.

14. The medical device of aspect 13, wherein the first conductor lead and the second conductor lead are configured to provide energy to the electrode and the first conductor lead and the thermocouple conductor are configured to produce a temperature dependent electrical response.

15. The medical device of aspect 9, further comprising a plurality of electrodes disposed in an array on the shaft distal portion.

16. The medical device of aspect 15, further comprising a temperature sensor associated with each electrode, the temperature sensor being selected from at least one of a thermocouple, thermistor, resistance temperature detector, electrical thermometer, and biomaterial thermometer.

17. The medical device of aspect 16, wherein the temperature sensor includes a printed lead electrically coupled to the temperature sensor.

18. A medical device, comprising:

an elongate shaft extending along a shaft longitudinal axis and comprising a shaft proximal portion and a shaft distal portion;
an ablation tip connected to the shaft distal portion, wherein the ablation tip is a conductive shell disposed over an end of the shaft distal portion;
a printed temperature sensor thermally coupled with the conductive shell, wherein the temperature sensor is a printed temperature sensor and is configured to sense a temperature of the conductive shell; and
a pair of leads that extend along the elongate shaft and are electrically coupled with the temperature sensor.

19. The medical device of aspect 18, wherein the temperature sensor is disposed on the shaft distal portion and is thermally coupled with an interior surface of the conductive shell.

20. The medical device of aspect 18, wherein the temperature sensor is disposed on an interior surface of the conductive shell.

21. The medical device of aspect 18, wherein the temperature sensor is one of a plurality of temperature sensors that are thermally coupled with the conductive shell.

22. The medical device of aspect 21, wherein the plurality of temperature sensors are thermocouples.

23. The medical device of aspect 18, wherein the plurality of temperature sensors are resistance temperature detectors.

24. The medical device of aspect 18, wherein the temperature sensor is printed on a substrate and the substrate is disposed on the conductive shell.

25. The medical device of aspect 24, wherein the substrate includes a three-dimensional profile.

26. The medical device of aspect 25, further comprising an electrode disposed on the substrate.

26. A medical device, comprising:

an elongate shaft extending along a shaft longitudinal axis and comprising a shaft proximal portion and a shaft distal portion;
a balloon disposed along the shaft distal portion; and
a plurality of temperature sensors disposed on the balloon.

27. The medical device of aspect 26, wherein the plurality of temperature sensors are resistance temperature detectors.

28. The medical device of aspect 26, wherein the temperature sensors are disposed on at least one of an interior surface and an exterior surface of the balloon.

29. The medical device of aspect 26, wherein the temperature sensors are concentrically disposed along the balloon about an outer most circumference of the ablation balloon.

30. A medical device, comprising:

an elongate shaft extending along a shaft longitudinal axis and comprising a proximal portion and a distal portion;
an elevated electrode disposed on the distal portion, wherein the elevated electrode is a spherical cap, the spherical cap includes a core formed from a first conductive material, and wherein an exterior of the core is coated with a second conductive material.

31. The medical device of aspect 30, wherein the second conductive material is more conductive than the first conductive material.

32. The medical device of aspect 31, wherein the first conductive material includes copper.

33. The medical device of aspect 32, wherein the second conductive material includes gold.

34. The medical device of aspect 33, wherein the spherical cap is hemispherical.

35. The medical device of aspect 34, wherein the first conductive material is formed in a hemispherical configuration.

36. The medical device of aspect 35, wherein the second conductive material uniformly coats an exterior of the first conductive material.

37. The medical device of aspect 30, wherein the distal portion includes an end effector, and wherein the elevated electrode is disposed on the end effector.

DETAILED DESCRIPTION

Electrodes and/or temperature sensors can be used to perform therapeutic and/or diagnostic functions in regards to the body. Electrodes and temperature sensors can be formed as separate elements on a medical device, which can be used to contact tissue associated with the body. Embodiments of the present disclosure can provide an electrode and temperature sensor that is incorporated into one element, thus allowing for the temperature sensor to measure a temperature at the site where the electrode is contacting the tissue. This can improve accuracy and can also decrease a cost of goods associated with the manufacture of the temperature sensor and electrode, by combining both the temperature sensor and electrode into one element. Additionally, embodiments of the present disclosure can provide for an electrode and/or temperature sensor that includes a three-dimensional profile. The three-dimensional profile of the electrode and/or the temperature sensor can improve contact between the electrode and/or temperature sensor and tissue of the body. For example, the three-dimensional profile can be of a raised height, which can cause the electrode and/or temperature sensor to contact the tissue before other portions of a medical device on which the electrode and/or temperature sensor are disposed. In some embodiments of the present disclosure, electrodes and/or temperature sensors can be arranged in arrays, each electrode and/or temperature sensor being connected to a processing unit via printed leads.

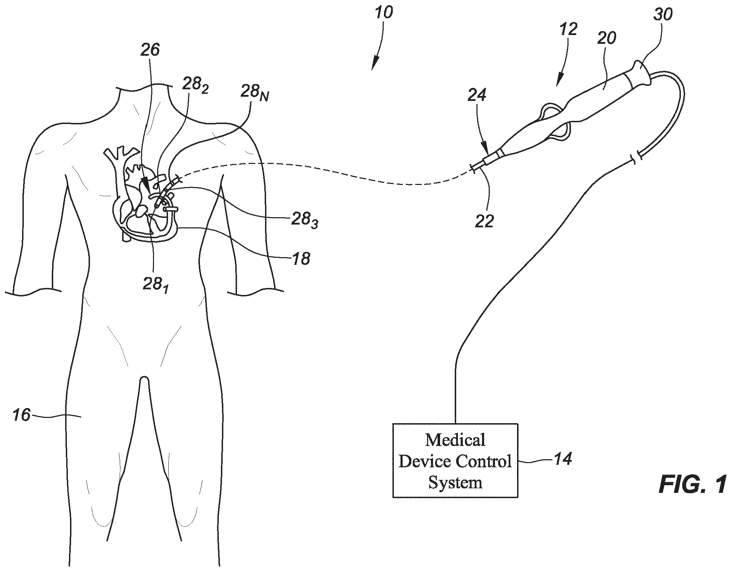

In some embodiments, and with reference to FIG. 1, a system 10 can include a medical device 12 and a medical device control system 14. The medical device 12 can include an elongate medical device such as, for example, a catheter or a sheath. For purposes of illustration and clarity, the description below will be limited to an embodiment wherein the medical device 12 comprises a catheter (e.g., catheter 12). It will be appreciated, however, that the present disclosure is not meant to be limited to such an embodiment, but rather in other exemplary embodiments, the medical device may comprise other elongate medical devices, such as, for example and without limitation, sheaths, introducers, guidewires, and the like.

With continued reference to FIG. 1, the catheter 12 can be configured to be inserted into a patient's body 16, and more particularly, into the patient's heart 18. The catheter 12 may include a handle 20, a shaft 22 having a proximal end portion 24 and a distal end portion 26, and one or more position sensors 28 mounted in or on the shaft 22 of the catheter 12. As used herein, "position sensor 28" or "position sensors 28" may refer to one or more position sensors $28_1$, $28_2$, $28_3$, . . ., $28_N$, as appropriate and as generally depicted. In an exemplary embodiment, the position sensors 28 are disposed at the distal end portion 26 of the shaft 22 and can be impedance based position sensors (e.g., electrodes) and/or magnetic based position sensors (e.g., a wound coil, as depicted and discussed in relation to Fig. 1B). For example, the position sensor $28_1$ can be a magnetic based position sensor and the position sensors $28_2$, $28_3$, . . ., $28_N$ can be impedance based position sensors. The catheter 12 may further include other conventional components such as, for example and without limitation, a temperature sensor, additional sensors or electrodes, ablation elements (e.g., ablation tip electrodes for delivering RF ablative energy, high intensity focused ultrasound ablation elements, etc.), and corresponding conductors or leads. These components can be electrically coupled with the medical device control system 14, which be a computing device and can include a memory and processor configured to execute instructions stored on the memory.

The shaft 22 can be an elongate, tubular, flexible member configured for movement within the body 16. The shaft 22 supports, for example and without limitation, sensors and/or electrodes mounted thereon, such as, for example, the position sensors 28, associated conductors, and possibly additional electronics used for signal processing and conditioning. The shaft 22 may also permit transport, delivery, and/or removal of fluids (including irrigation fluids, cryogenic ablation fluids, and bodily fluids), medicines, and/or surgical tools or instruments. The shaft 22 may be made from conventional materials such as polyurethane, and define one or more lumens configured to house and/or transport electrical conductors, fluids, or surgical tools. The shaft 22 may be introduced into a blood vessel or other structure within the body 16 through a conventional introducer. The shaft 22 may then be steered or guided through the body 16 to a desired location, such as the heart 18, using means well known in the art.

The position sensors 28 mounted in or on the shaft 22 of the catheter 12 may be provided for use in a variety of diagnostic and therapeutic purposes including, for example and without limitation, electrophysiological studies, pacing, cardiac mapping, and ablation. In an exemplary embodiment, one or more of the position sensors 28 are provided to perform a location or position sensing function. More particularly, and as will be described in greater detail below, one or more of the position sensors 28 are configured to provide information relating to the location (e.g., position and orientation) of the catheter 12, and the distal end portion 26 of the shaft 22 thereof, in particular, at certain points in time. Accordingly, in such an embodiment, as the catheter 12 is moved along a surface of a structure of interest of the heart 18 and/or about the interior of the structure, the position sensor(s) 28 can be used to collect location data points that correspond to the surface of, and/or other locations within, the structure of interest. These location data points can then be used for a number of purposes such as, for example and without limitation, the construction of surface models of the structure of interest.

For purposes of clarity and illustration, the description below will be with respect to an embodiment with a single position sensor 28. It will be appreciated, however, that in other exemplary embodiments, which remain within the spirit and scope of the present disclosure, the catheter 12 may comprise more than one position sensor 28 as well as other sensors or electrodes configured to perform other diagnostic and/or therapeutic functions. As will be described in greater detail below, the position sensor 28 can include a pair of leads extending from a sensing element thereof (e.g., a coil) that are configured to electrically couple the position sensor 28 to other components of the system 10, such as, for example, the medical device control system 14.

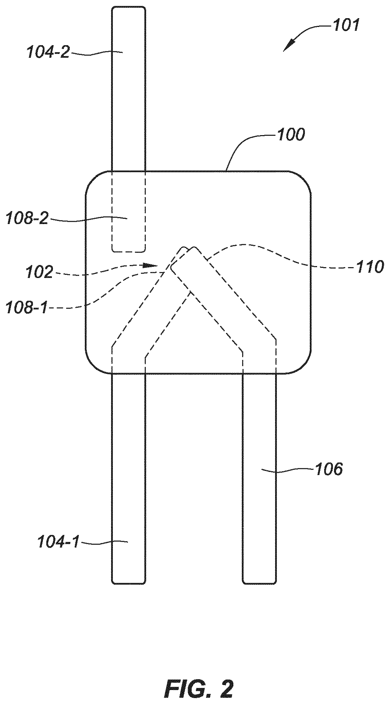

Fig. 2 is a diagrammatic top view of an electrode assembly 101 that includes an electrode 100 with a temperature sensor 102, in accordance with embodiments of the present disclosure. In some embodiments, the electrode 100 can be formed from a conductive material (e.g., gold, platinum-iridium, etc.) and can be disposed on a shaft (e.g., catheter shaft, introducer, etc.). As depicted in Fig. 2, the electrode 100 can be formed as a square, although the electrode 100 can be formed in other shapes, such as a circle, triangle, rectangle, etc. In some embodiments, the electrode 100 can be formed via printing. In some embodiments, the electrode 100 can be a spot electrode, ring electrode, etc. In some embodiments, the electrode 100 can be a sensing electrode, receiving electrical signals from a tissue and transmitting them to a computer for further analysis. In some embodiments, the electrode 100 can be an ablation electrode, where electrical signals are provided to the electrode 100, causing the electrode 100 to be heated.

In some embodiments, electrical traces can be disposed underneath the electrode 100, which can form the temperature sensor 102. In some embodiments, the temperature sensor 102 can be a trace temperature sensor, which includes conductive electrical traces that are formed from an additive and/or subtractive manufacturing process. In an example, a first conductor lead 104-1 and a second conductor lead 104-2 can be disposed underneath the electrode 100. Additionally, a thermocouple conductor 106 can be disposed underneath the electrode 100. For example, the first conductor lead 104-1, second conductor lead 104-2, and/or the thermocouple conductor 106 can be disposed between a shaft on which the electrode 100 is disposed and the electrode 100. The first conductor lead 104-1, second conductor lead 104-2, and the thermocouple conductor 106 can be disposed underneath the electrode 100 and/or within the electrode 100, in some embodiments. The first conductor lead 104-1, second conductor lead 104-2, and the thermocouple conductor 106 can be formed via printing in some embodiments.

In an example, the electrode 100 can be formed on top of the first conductor lead 104-1, second conductor lead 104-2, and the thermocouple conductor 106 and/or around the first conductor lead 104-1, second conductor lead 104-2, and the thermocouple conductor 106. For example, by forming the electrode 100 around the first conductor lead 104-1, second conductor lead 104-2, and the thermocouple conductor 106, the first conductor lead 104-1, second conductor lead 104-2, and the thermocouple conductor 106 can be disposed within the electrode 100. In some embodiments, the electrode can be formed via an additive process, such as chemical vapor deposition (CVD).

In some embodiments, the first conductor lead 104-1 and the thermocouple conductor 106 can be electrically coupled to one another. For example, as depicted in Fig. 2, a first conductor tip 108-1 of the first conductor lead 104-1 and a thermocouple tip 110 of the thermocouple conductor 106 can be coupled to one another to form the temperature sensor 102. In an example, the first conductor lead 104-1 and/or first conductor tip 108-1 and the thermocouple conductor 106 and/or thermocouple tip 110 can form a thermocouple junction, which comprises a junction between the first conductor lead 104-1 and/or first conductor tip 108-1 and the thermocouple conductor 106 and/or thermocouple tip 110. As depicted, a second conductor tip 108-1 of the second conductor lead 104-2 can be disposed adjacent to the connection between the first conductor lead 104-1 and the thermocouple conductor 106 and adjacent to the first conductor tip 108-1 and the thermocouple tip 110. In some embodiments, the thermocouple tip 110 of the thermocouple conductor 106 can be disposed on top of the first conductor tip 108-1 of the first conductor lead 104-1 to position the thermocouple tip 110 closer to a surface of the electrode 100. In some embodiments, this can provide a more responsive and/or more accurate temperature measurement of the surface of the electrode 100.

In some embodiments, the thermocouple conductor 106 can be formed from a type K, type J, type T or other type of material that can be used for construction of a thermocouple. For example, type K materials can include Nickel-Chromium/Nickel-Alumel; type J materials can include Iron/Constantan; and type T materials can include Copper/Constantan. In some embodiments, other materials such as Platinum Rhodium/Platinum can be used in the thermocouple conductor 106. In some embodiments, the thermocouple conductor 106 can be formed by printing the conductor. The thermocouple conductor 106 can have a thickness in a range from 0.0002 to 0.008 inches and can have a width in a range from 0.0002 to 0.008 inches, in some embodiments.

In some embodiments, power can be provided to the electrode 100 via the first conductor lead 104-1 and the second conductor lead 104-2. By providing power to the electrode 100 via the first conductor lead 104-1 and the second conductor lead 104-2, the electrode 100 can be heated to a first temperature. In an example, a therapy can be performed by placing the electrode 100 on a tissue, such as a tissue of the heart, and heating the electrode to a particular temperature by providing power via the first conductor lead 104-1 and the second conductor lead 104-2. For example, power can be provided to the electrode 100 via the first conductor lead 104-1 and the second conductor lead 104-2, causing the electrode 100 to be heated to the particular temperature. Control over the power provided to the electrode 100 from the first conductor lead 104-1 and the second conductor lead 104-2 can be modulated via the medical device control system 14 discussed in Fig. 1.

In some embodiments, the electrode 100 can serve as an ablation electrode and electricity can be transferred through the electrode 100 via the first conductor lead 104-1 and/or the second conductor lead 104-2. For example, in some embodiments, an external patch can be placed on a patient and electricity can be transferred from the external patch through the patient and to the electrode 100 via the first conductor lead 104-1 and/or the second conductor lead 104-2.

In some embodiments, a signal can be received via the electrode 100. In an example, the electrode 100 can be placed in contact with a tissue. The tissue can generate an electrical signal, which can be received by the electrode 100 and transferred to a computer (e.g., medical device control system 14) via the first conductor lead 104-1 and/or the second conductor lead 104-2.

In some embodiments, the first conductor lead 104-1 can be a multipurpose conductor. For example, the first conductor lead 104-1 can supply power to the electrode 100 and/or receive energy from the electrode 100 in the form of electrical signals generated by the heart and/or electricity transferred through the patient via the external patch. The first conductor lead 104-1 can also complete a circuit of the temperature sensor 102. For example, as a temperature of the electrode 100 changes and thus a temperature of the temperature sensor 102 changes, the thermocouple conductor 106 can convert the change in temperature to an electrical voltage, which can be analyzed to determine the temperature of the temperature sensor 102. For example, the temperature sensor 102 can be a thermocouple formed by the first conductor lead 104-1 and the thermocouple conductor 106. Although a thermocouple is given as an example of a temperature sensor herein, the temperature sensor can be any other type of temperature sensor, including thermistors, resistance temperature detectors, electrical thermometers, bimaterial thermometers, etc. In an example, thermistors can sense temperature based on resistance and can be formed via printing. Electrical thermometers can determine temperature as a result of a resistivity change due to temperature and can be formed via printing. Bimaterial thermometers can determine temperature via detection of mechanical motion based on different thermal expansion coefficients.

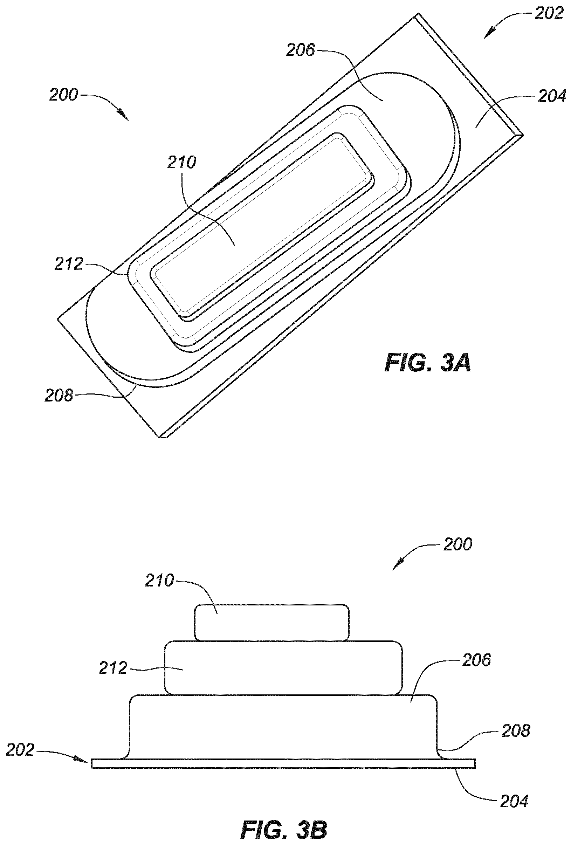

As a result of embodiments of the present disclosure, temperature at a tissue that comes into contact with the electrode 100 can be better measured. Furthermore, a cost of goods associated with the electrode 100 and associated temperature sensor can be reduced as a result of the incorporation of the electrode 100 and temperature sensor into one element and also through construction of the electrode and associated temperature sensor via additive manufacturing methods, as discussed herein. In some embodiments, the electrode and associated temperature sensor can be incorporated into a three-dimensional base, as further discussed in relation to Fig. 3A.

Fig. 3A is a diagrammatic top view of a three-dimensional electrode 200, in accordance with embodiments of the present disclosure. In some embodiments, the three-dimensional electrode 200 can include a three-dimensional base 202. The three-dimensional base 202 can be formed from a substrate that includes a planar base portion 204 and a three-dimensional profile portion 206. In an example, the substrate can be formed such that it defines a three-dimensional shape. For example, the substrate can initially be a planar piece of material, which can be pressed and/or constructed to form the three-dimensional profile portion 206 in the three-dimensional base 202. In some embodiments, a profile space can exist beneath the three-dimensional profile portion 206.

As depicted in Fig. 3A, the three-dimensional profile portion 206 can extend upwards, as better illustrated in Fig. 3B. The three-dimensional electrode 200 can include a perimeter interface 208 between the three-dimensional profile portion 206 and the planar base portion 204. As depicted in Fig. 3A, the perimeter interface 208 is oblong in shape. However, the perimeter interface 208 can be any shape, including circular, square, rectangular, polygonal, triangular, elliptical, etc. Although the planar base portion 204 is depicted, in some embodiments, there may be no planar base portion 204. For example, in some embodiments, the three-dimensional profile portion 206 can be formed such that the planar base portion 204 is not present and/or the planar base portion 204 can be trimmed from the three-dimensional profile portion 206. For instance, the planar base portion 204 can be trimmed from the three-dimensional profile portion 206 along the perimeter interface 208 or adjacent to the perimeter interface 208.

In some embodiments, the three-dimensional base 202 can be formed from a deformable material. For example, the three-dimensional base 202 can be formed from a metal, polymer, or other type of material, which can be deformed via application of heat and/or pressure to the material. In an example, if the three-dimensional base 202 is formed from a metal, the three-dimensional profile portion 206 can be formed by casting the three-dimensional base 202 and/or pressing the three-dimensional base 202 (e.g., via tool and die) to form the three-dimensional profile portion 206. In some embodiments where the three-dimensional base 202 is formed from a polymer, the polymer can be formed such that the three-dimensional profile portion 206 is formed in the three-dimensional base 202. For example, the polymer can be cast such that the three-dimensional base 202 includes the three-dimensional profile portion 206 and/or the polymer can be heated and/or pressure can be applied to the polymer to form the three-dimensional profile portion 206.

In some embodiments, the three-dimensional base 202 can be formed through an additive manufacturing process. For example, in some embodiments the three-dimensional base 202 can be formed by depositing material onto a mold through an additive process, such as printing, chemical vapor deposition, etc. In some embodiments, after the material has been deposited onto the mold, the material can be cured and the mold can be released from the material, thus forming the three-dimensional base 202. In some embodiments, the three-dimensional base can be formed via a subtractive process (e.g., laser etching, chemical etching, machining, etc.).

In some embodiments, an electrode 210 can be formed on top of the three-dimensional base 202. In an example, the electrode 210 can be formed from a metallic material, in some embodiments. For instance, the electrode 210 can be formed from gold, platinum, silver, etc. As depicted, the electrode 210 can be of an elongate rectangular shape in some embodiments. However, the electrode 210 can be of another shape, which can be circular, square, rectangular, polygonal, triangular, elliptical, pyramidal, hourglass, etc.

In some embodiments, a tie layer 212 can be disposed between the three-dimensional base 202 and the electrode 210, which can help to bond the electrode 210 to the three-dimensional base 202. The tie layer 212 can be formed from a conductive material such as a metal. In an example, the tie layer 212 can be formed from a metal such as nickel, sputtered chrome, etc. A dielectric layer can be disposed beneath the tie layer, insulating the electrode 210 and the dielectric layer from the three-dimensional base 202. In some embodiments, the electrode 210 can be formed as described in U.S. Application no. 15/331,562, which is hereby incorporated by reference as though fully set forth herein.

As discussed herein, an electrode and/or temperature sensor can be formed with a three-dimensional profile using additive methods. In some embodiments, however, the electrode and/or temperature sensor can be formed with a three-dimensional profile via pressing a substrate blank, as discussed below.

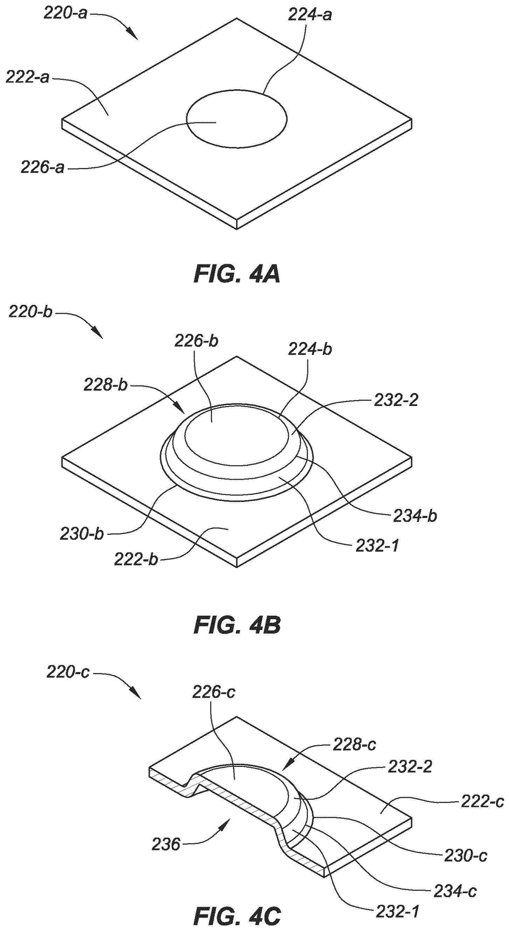

Fig. 4A is an isometric view of a substrate blank 220-a for use in the formation of a three-dimensional electrode as further depicted in Figs. 4A and 4B in accordance with embodiments of the present disclosure. In some embodiments, the substrate blank 220-a can be formed from a planar piece of material 222-a. As depicted, the planar piece of material 222-a is square in shape, although the planar piece of material 222-a can be of another shape (e.g., circular, triangular, rectangular, oblong, etc.). A sketch 224-a is depicted on the surface of the planar piece of material 222-a, which in some embodiments can outline a face 226-a where an electrode can be disposed. For example, the face 226-a contained by the sketch 224-a can be prepared (etched, coated, etc.) in order to prepare the area for application of one or more layers of coatings, which can form an electrode. For example, an electrode can be disposed on the face 226-a contained by the sketch 224-a via an additive manufacturing process (e.g., printing, vapor deposition, etc.), as discussed herein.

In some embodiments, a three-dimensional profile portion 228-b, 228-c (Figs. 4B, 4C) can be formed in the substrate blank 220-a, as previously discussed. The three-dimensional profile portion 228-b, 228-c can extend upwards from the otherwise planar piece of material 222-a and can be formed in the planar piece of material 222-a, as previously discussed and further depicted in Figs. 4B and 4C.

Fig. 4B is an isometric view of the substrate blank 220-b depicted in Fig. 4A with a three-dimensional profile portion 228-b formed in the substrate blank 220-b. In an example, the substrate blank 220-b can be pressed and/or constructed to form the three-dimensional profile portion 228-b, as previously discussed. A perimeter interface 230-b can exist between an interface of the three-dimensional profile portion 228-b and the planar piece of material 222-b. As depicted in Fig. 4B, the perimeter interface 230-b can be circular in shape, however, the perimeter interface 230-b can be any shape, including square, rectangular, polygonal, triangular, elliptical, oblong, etc. Although the planar piece of material 222-b is depicted, in some embodiments, the planar piece of material 222-b can be trimmed from the three-dimensional profile portion 228-b. For example, the planar piece of material 222-b can be trimmed from the three-dimensional profile portion 228-b along the perimeter interface 230-b or adjacent to the perimeter interface 230-b.

In some embodiments, the three-dimensional profile portion 228-b can have a stepped profile. For example, as depicted in Fig. 4B, the three-dimensional profile portion 228-b can have a first stepped profile portion 232-1 and a second stepped profile portion 232-2, which can be separated by a profile interface 234-b. For example, the first profile portion 232-1 can extend upward from the planar piece of material 222-b from the perimeter interface 230-b to the profile interface 234-b. The profile interface 234-b can be a stepped portion from which a second stepped profile portion 232-2 extends upward. The second profile portion 232-2 can extend upwards from the profile interface 234-b to the face 226-b contained by sketch 224-b.

In some embodiments, the upwardly extending walls of the first profile portion 232-1 and/or the second profile portion 232-2 can extend upwards at an angle that is perpendicular to the planar piece of material 222-b. In some embodiments and as depicted, the walls of the first profile portion 232-1 and/or the second profile portion 232-2 can extend upwards at an angle that is between an angle that is parallel with the planar piece of material 222-b and an angle that is perpendicular to the planar piece of material 222-b. In an example, the walls of the first profile portion 232-1 and the second profile portion 232-2 can be frustoconical in shape.

In some embodiments, an electrode can be disposed on the face 226-b contained by the sketch 224-b. For example, an electrode can be formed on a portion of the face 226-b via an additive manufacturing process, as previously discussed herein. By forming the electrode on the portion of the face 226-b, the electrode can be elevated from a surface of the planar piece of material 222-b. This can create a stand-off to a geometry of a medical device on which the electrode is disposed, allowing for a better contact between the electrode disposed on the face 226-b and tissue.

In some embodiments, the electrode can be formed on the face 226-b, the first profile portion 232-1, the profile interface 234-b, and/or the second profile portion 232-2. For example, the electrode can be disposed on one or more portions of the three-dimensional profile portion 228-b. In an example, the electrode can be disposed on portions of the three-dimensional profile portion 228-b that extend upward from the planar piece of material 222-b. In some embodiments, the electrode can be formed on the face 226-b, the first profile portion 232-1, the profile interface 234-b, and/or the second profile portion 232-2 via an additive manufacturing process.

Fig. 4C is an isometric cross-sectional view of the substrate blank 220-c depicted in Fig. 4B, in accordance with embodiments of the present disclosure. As discussed in relation to Fig. 4B, the substrate blank 220-c includes the planar piece of material 222-c, from which extends the three-dimensional profile portion 228-c. The perimeter interface 230-c can exist between an interface of the three-dimensional profile portion 228-c and the planar piece of material 222-c. In some embodiments, the three-dimensional profile portion 228-c can have a stepped profile. For example, as depicted in Fig. 4C, the three-dimensional profile portion 228-c can have the first stepped profile portion 232-1 and the second stepped profile portion 232-2, which can be separated by the profile interface 234-c. The second stepped profile portion 232-2 can extend upwards from the profile interface 234-c to the face 226-c. As depicted, a profile space 236 can be defined beneath the three-dimensional profile portion 228-c.

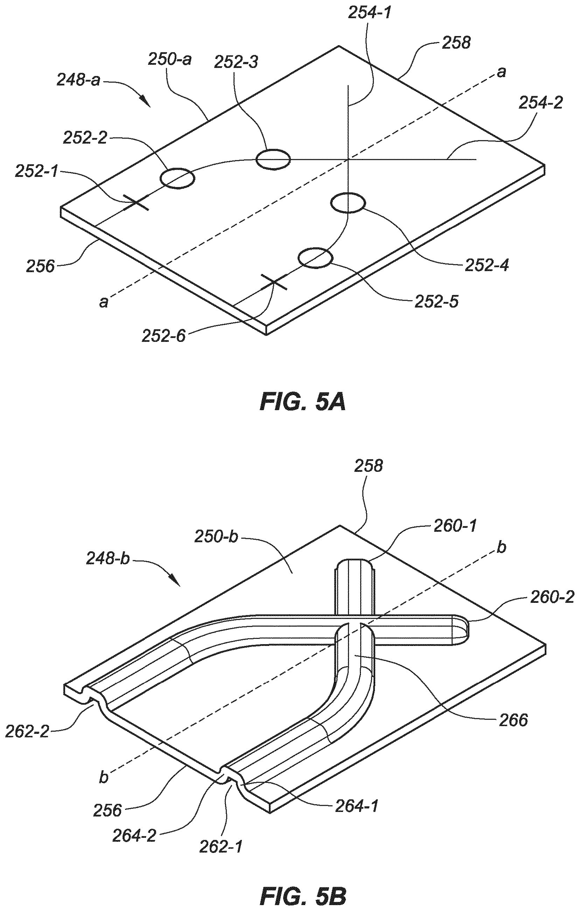

As discussed in relation to Fig. 2, in some embodiments, electrical traces can be disposed underneath an electrode disposed on the face 226-a, 226-b, 226-c depicted in Figs. 4A to 4C, respectively, which can form a temperature sensor. In some embodiments, the temperature sensor can be a trace temperature sensor, which includes conductive electrical traces that are formed from an additive and/or subtractive manufacturing process. In some embodiments, the three-dimensional profile portion 228-b, 228-c can be used as part of an array and/or can help a device conform to different tissue geometries. Fig. 5A is an isometric view of a substrate blank 248-a for use in the formation of a three-dimensional electrode as further depicted in Fig. 5B, in accordance with embodiments of the present disclosure. In some embodiments, the substrate blank 248-a can be formed from a planar piece of material 250-a. As depicted, the planar piece of material 250-a is rectangular in shape and can extend along a planar axis defined by line aa, although the planar piece of material 250-a can be of another shape (e.g., circular, triangular, square, oblong, etc.).

One or more sketches can be made on the planar piece of material 250-a, which in some embodiments can outline and/or define an area where an electrode can be disposed. For example, sketches 252-1, 252-2, 252-3, 252-4, 252-5, 252-6, hereinafter referred to in the plural as sketches 252, can be made on the planar piece of material 250-a. For example, as previously discussed herein, the area contained and/or defined by the sketches 252 can be prepared (etched, coated, etc.) in order to prepare the area for application of one or more layers of coatings, which can form an electrode. For example, an electrode can be disposed in the area contained by the sketch 252 via an additive manufacturing process, as discussed herein.

In some embodiments, a three-dimensional profile portion can be formed in the substrate blank 250-a, as previously discussed. The three-dimensional profile portion, further depicted and discussed in relation to Fig. 5B, can extend upwards from the otherwise planar piece of material 250-a and can be formed in the planar piece of material 250-a. Three-dimensional profile sketches 254-1, 254-2 are depicted on a surface of the substrate blank 250-a. The three-dimensional profile sketches 254-1, 254-2 can indicate where the three-dimensional profile portion can be formed in the substrate blank 250-a. As depicted, the first three-dimensional profile sketch 254-1 can extend from a first edge 256 of the substrate blank 250-a towards a second edge 258 of the substrate blank 250-a. In some embodiments, the first three dimensional profile sketch 254-1 can extend from the first edge 256 parallel with the longitudinal axis aa, before crossing over the longitudinal axis aa at an angle. In some embodiments, the second three dimensional profile sketch 254-2 can extend from the first edge 256 parallel with the longitudinal axis aa, before crossing over the longitudinal axis aa at an angle. In an example, the first and second three-dimensional profile sketches 254-1, 254-2 can intersect on the longitudinal axis aa.

Fig. 5B is an isometric view of the substrate blank 250-b depicted in Fig. 5A with three-dimensional profile portions 260-1, 260-2 formed in the substrate blank 250-b, in accordance with embodiments of the present disclosure. In some embodiments, the substrate blank 250-b can be pressed and/or constructed to form the three-dimensional profile portions 260-1, 260-2. The three-dimensional profile portions 260-1, 260-2 can be formed along the three-dimensional profile sketches 254-1, 254-2.

In some embodiments, the first three dimensional profile portion 260-1 can extend from the first edge 256 parallel with the longitudinal axis bb and toward the second edge, before crossing over the longitudinal axis bb at an angle. In some embodiments, the second three dimensional profile portion 260-2 can extend from the first edge 256 parallel with the longitudinal axis bb and toward the second edge 258, before crossing over the longitudinal axis bb at an angle. In an example, the first and second three-dimensional profile portions 260-1, 260-2 can intersect on the longitudinal axis aa. As depicted, in Fig. 5B, the three-dimensional profile portions 260-1, 260-2 can define profile spaces 262-1, 262-2 beneath the three-dimensional profile portions 260-1, 260-2.

As depicted, the three-dimensional profile portions 260-1, 260-2 can include profile walls and faces that form the three-dimensional portions 260-1, 260-2. For example, with respect to the first three-dimensional profile portion 260-1, this profile portion can include a first profile wall 264-1 and a second profile wall 264-2 that extend from a surface of the substrate blank 250-b. A profile face 266 can extend across the top of each profile wall 264-1, 264-2, thus defining the profile space 262-1.

In some embodiments, although not depicted, one or more electrodes can be disposed and/or formed on the sketches 252. For example, an electrode can be formed on a portion of the three-dimensional profile portions 260-1, 260-2. The electrode can be disposed along the three-dimensional profile portions 260-1, 260-2 in one or more discrete locations (e.g., spot electrodes). In some embodiments, the electrodes can be formed along an entire length and/or portions of the length of each one of the three-dimensional profile portions 260-1, 260-2. Although Figs. 4A to 5B depict particular shapes of three-dimensional profiles, the shapes are not so limited and the three-dimensional profile portions can be of any shape, as discussed herein.

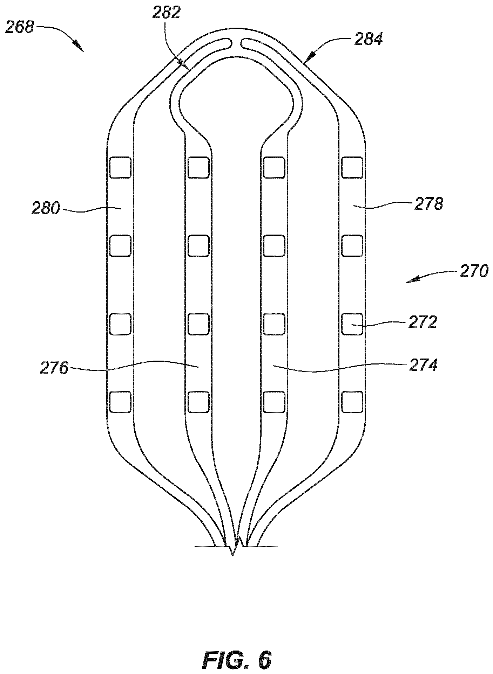

As discussed in relation to Fig. 2, in some embodiments, electrical traces can be disposed underneath an electrode disposed on the three-dimensional profile portions 260-1, 260-2 depicted in Figs. 5A to 5C, respectively, which can form a temperature sensor. In some embodiments, the temperature sensor can be a trace temperature sensor, which includes conductive electrical traces that are formed from an additive and/or subtractive manufacturing process. The electrodes and/or temperature sensors and/or the three dimensional base, as discussed above, can be included on a medical device, as depicted and described in relation to Figs. 6 to 7B, for example. Fig. 6 is a top view of a high density electrode mapping catheter 68, according to various embodiments of the present disclosure. In some embodiments, the high density electrode mapping catheter 268 can include a flexible tip portion 270 that forms a flexible array of microelectrodes 272. In some embodiments, the high density electrode mapping catheter 270 can include a catheter shaft, not depicted, from which the flexible tip portion 270 extends.

The flexible tip portion 270 can form a planar array (or 'paddle' configuration) of microelectrodes 274, which comprises four side-by-side, longitudinally-extending arms 274, 276, 278, 280, which can form a flexible framework on which the microelectrodes 272 are disposed. The four microelectrode-carrier arms comprise a first outboard arm 274, a second outboard arm 276, a first inboard arm 278, and a second inboard arm 280. These arms can be laterally separated from each other.

Each of the four arms can carry a plurality of microelectrodes 272. For example, each of the four arms can carry microelectrodes 272 spaced along a length of each of the four arms. Although the high density electrode mapping catheter 268 depicted in Fig. 6 includes four arms, the high density electrode mapping catheter 268 could comprise more or fewer arms. Additionally, while the high density electrode mapping catheter 268 depicted in Fig. 6 includes 16 electrodes (e.g., 4 microelectrodes on each of the first outboard arm 278 and second outboard arm 280 and 4 microelectrodes on each of the first inboard arm 274 and second inboard arm 276), the catheter can include more or fewer than 16 electrodes. In addition, the first outboard arm 278 and second outboard arm 280 can include more or fewer than 4 microelectrodes and the first inboard arm 274 and second inboard arm 276 can include more or fewer than 4 microelectrodes).

In some embodiments, the microelectrodes 272 can be used in diagnostic, therapeutic, and/or mapping procedures. For example and without limitation, the microelectrodes 272 can be used for electrophysiological studies, pacing, cardiac mapping, and ablation. In some embodiments, the microelectrodes 272 can be used to perform unipolar or bipolar ablation. This unipolar or bipolar ablation can create specific lines or patterns of lesions. In some embodiments, the microelectrodes 272 can receive electrical signals from the heart, which can be used for electrophysiological studies. In some embodiments, the microelectrodes 272 can perform a location or position sensing function related to cardiac mapping. Additional details on one type of planar electrode array are discussed in relation to U.S. Application no. 15/331,562, which is incorporated by reference as though fully set forth herein.

In some embodiments, the microelectrodes 272 can include a temperature sensor such as that depicted and discussed in relation to Figs. 2 to 3B. In an example, a temperature sensor can be disposed underneath one or more of the microelectrodes 272 and/or formed within one or more of the microelectrodes 272. The temperature sensor can thereby measure a temperature of one or more of the microelectrodes 272. In some embodiments, one temperature sensor can be associated with each microelectrode 272. For example, one temperature sensor can be disposed beneath, within, and/or on top of each microelectrode 272. In some embodiments, the microelectrodes 272 can have a surface area of 0.2 millimeters$^2$, which can be a size associated with a diagnostic electrode, although the microelectrodes 272 can have a surface area that is greater than or smaller than 0.2 millimeters$^2$. In some embodiments, an ablation electrode can be of a larger size than 0.2 millimeters$^2$.

In some embodiments, the microelectrodes 272 can be three-dimensional electrodes, such as those discussed in relation to Figs 4A to 5B. For example, the microelectrodes 272 can extend upwards from a surface of the flexible tip portion 270. The microelectrodes 272 can be formed as discussed in relation to Figs. 4A to 5B. In an example, printed sensors (e.g., thermocouples, resistance temperature detectors, etc.) can be formed underneath and/or within the microelectrodes 272.

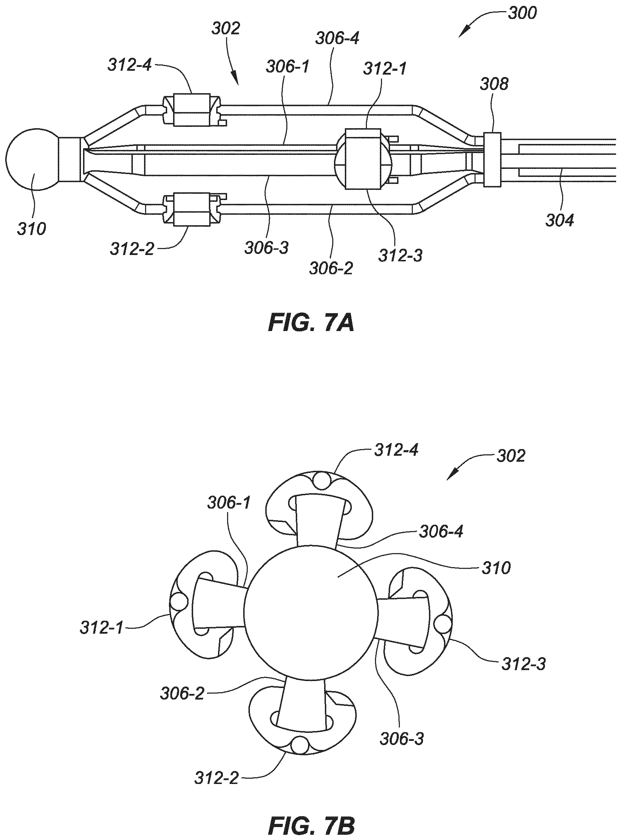

The embodiments disclosed herein, for example, those discussed in relation to Figs. 2 to 5B can be used in a variety of medical devices. For example, the embodiments disclosed herein can be used in a device similar to the EnligHTN™ Multi-Electrode Renal Denervation System, produced by St. Jude Medical, further discussed in U.S. Application no. 14/258,407, which is incorporated by reference as though full set forth herein. Fig. 7A is a side view of an exemplary renal denervation catheter 300, as discussed above, according to various embodiments of the present disclosure. Fig. 7B is an end view of a renal denervation catheter 300, according to various embodiments of the present disclosure. In some embodiments, the renal denervation catheter 300 can include a radial ablation tip 302, which is connected to a distal end of a catheter shaft 304. The renal denervation catheter 300 can be similar to the EnligHTN™ Multi-Electrode Renal Denervation System, produced by St. Jude Medical, further discussed in U.S. Application no. 14/258,407, which is incorporated by reference as though full set forth herein. The radial ablation tip 302 can include a plurality of radially expanding members 306-1, 306-2, 306-3, 306-4 that extend distally and axially from a connector 308, which is attached to the distal end of the shaft 304. In some embodiments, a distal end of each of the radially expanding members 306-1, 306-2, 306-3, 306-4 can be connected to a catheter tip 310. In some embodiments, as the shaft 304 can be extended or retracted in relation to the catheter tip 310, causing the radially expanding members 306-1, 306-2, 306-3, 306-4 to radially expand or radially retract.

In some embodiments, each one of the radially expanding members 306-1, 306-2, 306-3, 306-4 can include an electrode 312-1, 312-2, 312-3, 312-4 disposed thereon. As the radially expanding members 306-1, 306-2, 306-3, 306-4 expand, the electrode 312-1, 312-2, 312-3, 312-4 can contact a lumen in which the radial ablation tip 302 is disposed. For example, the radial ablation tip 302 can be disposed in a renal artery and the radial ablation tip 302 can be expanded to cause the electrodes 312-1, 312-2, 312-3, 312-4 to contact an inner wall of the renal artery. Upon contact with the inner wall, the electrodes 312-1, 312-2, 312-3, 312-4 can perform a sensing and/or therapeutic function. For example, the electrodes 312-1, 312-2, 312-3, 312-4 can sense electrical signals passing along the renal artery and/or can perform an ablation to the renal artery.

In some embodiments, the electrodes 312-1, 312-2, 312-3, 312-4 can include a temperature sensor such as that depicted and discussed in relation to Figs. 2 to 3B. In an example, a temperature sensor can be disposed underneath one or more of the electrodes 312-1, 312-2, 312-3, 312-4 and/or formed within one or more of the electrodes 312-1, 312-2, 312-3, 312-4. The temperature sensor can thereby measure a temperature of one or more of the electrodes 312-1, 312-2, 312-3, 312-4.

In some embodiments, the electrodes 312-1, 312-2, 312-3, 312-4 can be three-dimensional electrodes, such as those discussed in relation to Figs. 4A to 5B. For example, the electrodes 312-1, 312-2, 312-3, 312-4 can extend upwards from a surface of each one of the radially expanding members 306-1, 306-2, 306-3, 306-4. The electrodes 312-1, 312-2, 312-3, 312-4 can be formed as discussed in relation to Figs. 2 to 5B.

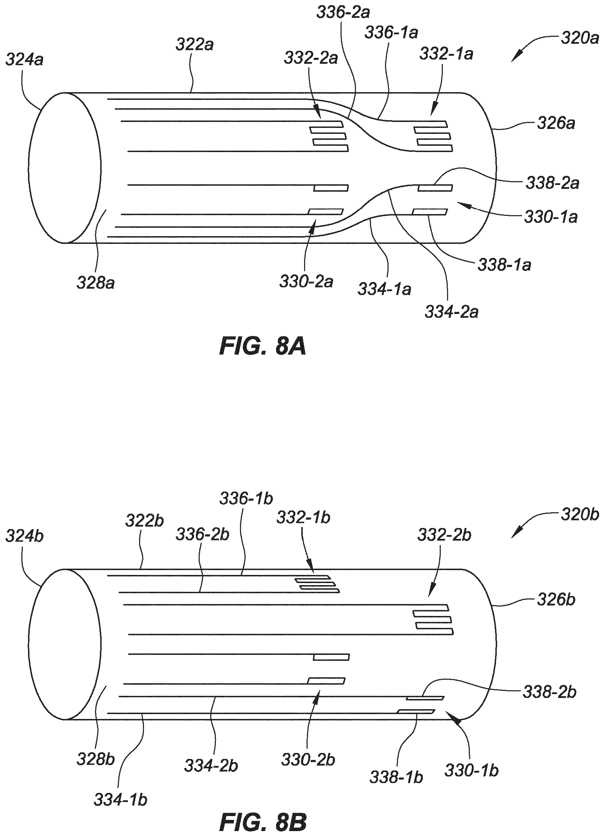

Fig. 8A is an isometric side view of a temperature enabled catheter tube 320a with axially aligned temperature sensors 330-1a, 330-2a, 332-1a, 332-2a, in accordance with embodiments of the present disclosure. In some embodiments, the temperature enabled catheter tube 320a can include a catheter tube 322a. The catheter tube 322a can extend along a longitudinal axis and can include a proximal end 324a and a distal end 326a. In some embodiments, the catheter tube 322a can define a central lumen that extends through the catheter tube and/or can be solid. The catheter tube 322a can be a catheter shaft and/or a portion of a catheter shaft in some embodiments. In some embodiments, the catheter tube 322a can be connected to a catheter shaft (e.g., a distal end of the catheter shaft). The catheter tube 322a can include a plurality of temperature sensors 330-1a, 330-2a, 332-1a, 332-2a, in some embodiments. The catheter tube 322a can be a portion of a catheter shaft and/or connected to a portion of a catheter shaft. In some embodiments, the catheter tube 322a can have a proximal end 324a and a distal end 326a, and can extend along a longitudinal axis and can define a lumen that extends therethrough. In some embodiments, the temperature sensors 330-1a, 330-2a, 332-1a, 332-2a can be disposed on an exterior surface 328a of the catheter tube 322a.

The temperature sensors 330-1a, 330-2a, 332-1a, 332-2a can be printed on the exterior surface 328a, as further discussed below. In some embodiments, the temperature sensors 330-1a, 330-2a, 332-1a, 332-2a can be printed on a substrate (e.g., film) and the film can be disposed on the exterior surface 328a. In an example, the temperature sensors 330-1a, 330-2a, 332-1a, 332-2a can be disposed in a particular array on the catheter tube 322a. In an example, the temperature sensors 330-1a, 330-2a are disposed on the catheter tube 322a such that they are axially aligned with one another. Likewise, the temperature sensors 332-1a, 332-2a are disposed on the catheter tube 322a such that they are axially aligned with one another.

Although four temperature sensors 330-1a, 330-2a, 332-1a, 332-2a are depicted, any number of temperature sensors can be disposed on the catheter tube 322a. For example, a number of temperature sensors 330-1a, 330-2a, 332-1a, 332-2a disposed on the catheter tube 322a can be in a range from 1 to 100 temperature sensors 330-1a, 330-2a, 332-1a, 332-2a. In some embodiments, the temperature sensors 330-1a, 330-2a, 332-1a, 332-2a can be equally distributed about the exterior surface 328a of the catheter tube 322a and/or arranged in particular patterns about the exterior surface 328a to enable a temperature profile to be constructed based on readings from the various temperature sensors.

In some embodiments, a size of each one of the temperature sensors 330-1a, 330-2a, 332-1a, 332-2a with respect to a surface area of a tip of a catheter can be approximately one percent of the total tip area. For example, where the tip of the catheter has a dimension of 2 millimeters by 4 millimeters (e.g., 8 millimeters$^2$), each one of the temperature sensors 330-1a, 330-2a, 332-1a, 332-2a can have a surface area that is 1 percent of the total 8 millimeters$^2$ surface area. In some embodiments, the temperature sensors 330-1a, 330-2a, 332-1a, 332-2a can be disposed on the surface of the tip of the catheter with a density of 1 temperature sensor per 1 to 4 millimeters$^2$ of surface area. In some embodiments, the tip of the catheter can include preferably 1 temperature sensor per 1.55 millimeters$^2$ of surface area.

In some embodiments, the temperature sensors 330-1a, 330-2a can be thermocouples. As diagrammatically represented, the temperature sensors 330-1a, 330-2a are thermocouples. In an example, the thermocouples 330-1a, 330-2a can be connected to a processing unit (e.g., computer) via a first lead and a second lead. For example, with reference to thermocouple 330-1a, the thermocouple 330-1a can be connected to the processing unit via a first lead 334-1a and a second lead 334-2a. A first thermocouple element 338-1a can be electrically coupled with the first lead 334-1a and a second thermocouple element 338-2a can be electrically coupled with the second lead 334-2a. As further discussed below, the first thermocouple element 338-1a can be electrically coupled with the second thermocouple element 338-2a. The first thermocouple element 338-1a and the second thermocouple element 338-2a can be formed from dissimilar metals and one of the metals can be a type of metal that produces a temperature dependent electrical response (e.g., Seebeck effect).

In some embodiments, the temperature sensors 332-1a, 332-2a can be resistance temperature detectors (RTDs), as diagrammatically represented. In an example, the RTDs 332-1a, 332-2a can be connected to a processing unit (e.g., computer) via a first lead and a second lead. For example, with reference to RTD 332-1a, the RTD 332-1a can be connected to the processing unit via a first lead 336-1a and a second lead 336-2a.

Direct-write electronic additive manufacturing, such as aerosol jet, micropen and/or ink-jet application of conductive traces and dielectrics, and materials such as high conductivity inks, can be used in embodiments of the present disclosure to enable the replacement of higher cost/labor intensive EP catheter components. These may include elements such as conductors and rings, but also more complicated elements such as temperature sensors, magnetic pick-up coils and force sensors. For example, temperature sensors such as the thermocouples 330-1a, 330-2a and/or the RTDs 332-1a, 332-2a can be disposed on the EP catheter components (e.g., catheter tube 322a).

Printed temperature sensors such as those discussed in Honda et. Al. "Printed Wearable Temperature Sensors for Health Monitoring", Sensor IEEE, 2014 can be included on the catheter components. In general, the temperature sensing array can have a temperature sensing element, conducting lead, and processing electronics, which can analyze a signal generated by the temperature sensing element.

The location and distribution of temperature sensors in the array may be optimized for prediction of both contact area and to provide initial / boundary conditions for thermal modeling of lesion depth, size etc. The temperature sensing element can be resistance based (e.g., RTD) or voltage based (e.g., thermocouple).

The process used to print the temperature sensor and associated leads onto the catheter shaft, tip or balloon can include processes for direct write printing of electronics including aerosol jet, micro-dispensing (micropen), and ink jet. Although direct write printing may be preferred, screen print and plating may also be used.

For resistance temperature detectors (RTD), these are typically comprised of a metallic trace printed in a serpentine geometry. The impedance of the RTD should be significantly higher than the leads (i.e. measured resistance predominately across sensor). The temperature dependence of the resistivity generally has two components. The first is the inherent temperature dependence of the metal bulk resistivity. The second contribution arises from the thermal expansion coefficient of the sensor construction (strain dependence of resistance). For an RTD, the relationship between a change in resistance ($\Delta R$) and temperature is given by:

$$\Delta R = R_o\, \alpha\, \Delta T$$

where $R_o$, is the resistance at temperature $T_o$, $\Delta T = T - T_o$, and $\alpha$ is the temperature coefficient of resistance.

For most conducting metals, the temperature coefficient is approximately 0.004/°C. As an example, a 1000 ohm thermistor will have a 4 ohm increase in resistance for every degree Celsius change.

For an RTD the temperature sensitive composition can be any material which is electrically conducting and printable thru direct write printing techniques. Examples of these can include conducting inks such as those based on silver, gold, copper, platinum and carbon, to name a few. Metals can be preferred, and platinum can be particularly preferred due to the relatively high temperature coefficient and reduced sinter-ability, which can enable higher impedance elements.

The geometry of the RTD resistive formulation can be optimized for temperature sensitivity and signal stability. For thermocouples, the temperature sensing element can be comprised of a printed union of two dissimilar metals known to produce a temperature dependent open circuit voltage (Seebeck effect). These can include metal combinations of standard thermocouples including types E, 3, K, R, S and T. In addition, metal combinations can also include less conventional metal combinations that are more amenable to direct write printing.

A preferred metal combination for a direct write printed thermocouple can be platinum and gold. For thermocouples, the processing electronics can also include the necessary temperature compensation for any additional dissimilar metal combinations within the circuitry.

Fig. 8B is an isometric side view of a temperature enabled catheter tube 320b with axially offset temperature sensors 330-1b, 330-2b, 332-1b, 332-2b, in accordance with embodiments of the present disclosure. In contrast to Fig. 8A, the temperature sensors 330-1b, 330-2b, 332-1b, 332-2b can be axially offset with respect to one another. In some embodiments, the temperature enabled catheter tube 320b can include a catheter tube 322b. The catheter tube 322b can extend along a longitudinal axis and can include a proximal end 324b and a distal end 326b. In some embodiments, the catheter tube 322b can define a central lumen that extends through the catheter tube and/or can be solid. The catheter tube 322b can be a catheter shaft and/or a portion of a catheter shaft in some embodiments. In some embodiments, the catheter tube 322b can be connected to a catheter shaft (e.g., a distal end of the catheter shaft).

The catheter tube 322b can include a plurality of temperature sensors 330-1b, 330-2b, 332-1b, 332-2b, in some embodiments. The catheter tube 322b can be a portion of a catheter shaft and/or connected to a portion of a catheter shaft. In some embodiments, the catheter tube 322b can have a proximal end 324b and a distal end 326b, and can extend along a longitudinal axis and can define a lumen that extends therethrough. In some embodiments, the temperature sensors 330-1b, 330-2b, 332-1b, 332-2b can be disposed on an exterior surface 328b of the catheter tube 322b.

In some embodiments, the temperature sensors 330-1b, 330-2b can be thermocouples. In an example, the thermocouples 330-1b, 330-2b can be connected to a processing unit (e.g., computer) via a first lead and a second lead. For example, with reference to thermocouple 330-1b, the thermocouple 330-1b can be connected to the processing unit via a first lead 334-1b and a second lead 334-2b. A first thermocouple element 338-1b can be electrically coupled with the first lead 334-1b and a second thermocouple element 338-2b can be electrically coupled with the second lead 334-2b. As further discussed below, the first thermocouple element 338-1b can be electrically coupled with the second thermocouple element 338-2b. Likewise, the temperature sensors 332-1b, 332-2b can be connected to the processing unit via leads 336-1b, 336-2b.

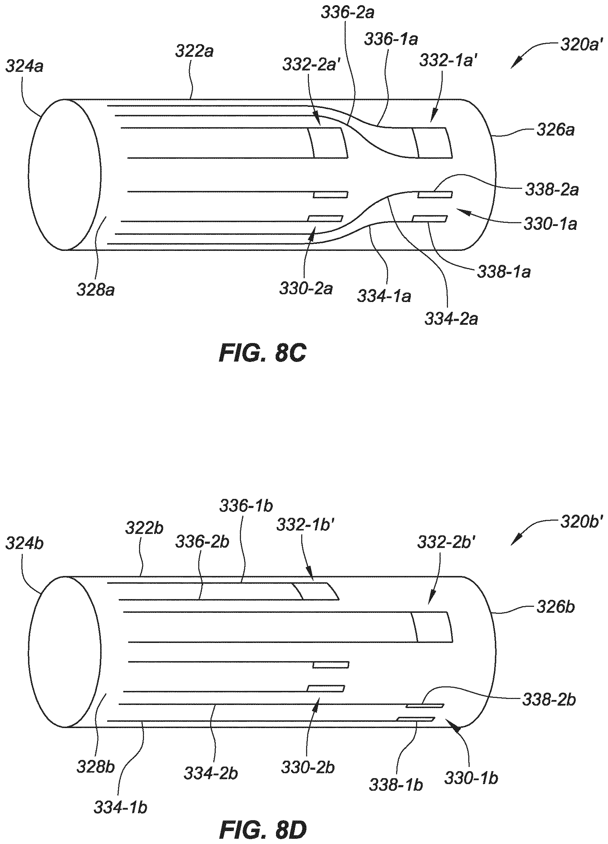

Figs. 8C and 8D are isometric side views of temperature enabled catheter tube 320a', 320b' with temperature sensors 330-1a, 330-2a, 332-1a', 332-2a', 330-1b, 330-2b, 332-1b', 332-2b', in accordance with embodiments of the present disclosure. Figs. 8C and 8D include features that are similar to and/or the same as those discussed in Figs. 8A and 8B, as denoted by the common numbering. However, as depicted, in Figs. 8C and 8D, the temperature sensors 332-1a', 332-2a', 332-1b', 332-2b' may not be disposed in a serpentine fashion, such as that depicted in Figs. 8A and 8B. For example, in some embodiments, the temperature sensors 332-1a', 332-2a', 332-1b', 332-2b' are depicted as being disposed in a square configuration. However, the temperature sensors 332-1a', 332-2a', 332-1b', 332-2b' can be disposed in other shaped configurations, in some embodiments. For example, the temperature sensors 332-1a', 332-2a', 332-1b', 332-2b' can be disposed in a triangular configuration, rectangular configuration, circular configuration, oval configuration, etc.

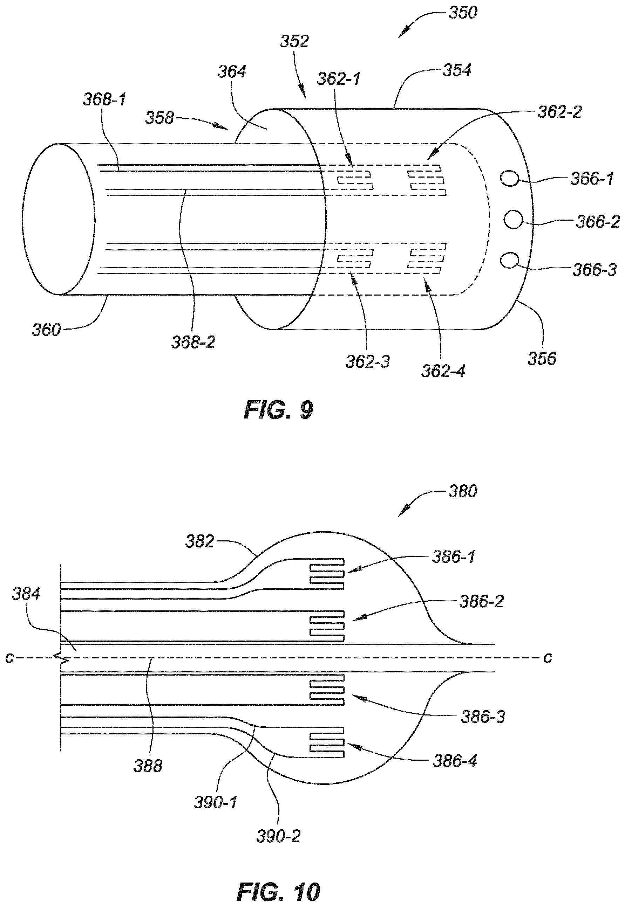

Fig. 9 is a diagrammatic isometric view of a temperature enabled catheter ablation tip 350, in accordance with embodiments of the present disclosure. The temperature enabled catheter ablation tip 350 can include an ablation tip 352. In some embodiments, the ablation tip 352 can be a radio frequency ablation tip. The ablation tip 352 can include a conductive shell that is formed from an electrically conductive material (e.g., metal) that includes a hollow cylindrical body 354 and a domed tip 356. The ablation tip 352 can include those features as discussed in relation to U.S. Application no. 15/088,052 and U.S. Application no. 14/911,474, which are hereby incorporated by reference as though fully set forth herein.

In some embodiments, the hollow cylindrical body 354 can define a lumen 358 in which a member (e.g., elongate tubular member, catheter tube 360) can be inserted. In some embodiments, the member can be any number of devices. In an example, the member can be an elongate member, such as an elongate tubular member that includes a fluid lumen, etc. However, for purposes of discussion, the member is discussed as a catheter tube 360 herein. In some embodiments, the catheter tube 360 can include features that are similar or the same as those discussed in relation to Fig. 8. For example, the catheter tube 360 can include a plurality of temperature sensors 362-1, 362-2, 362-3, 362-4, hereinafter referred to in the plural as temperature sensors 362, disposed in an array on an exterior surface of the catheter tube 360. In some embodiments, the temperature sensors 362 can be disposed next to an interior surface of the ablation tip 352. For example, in some embodiments, the diameter of the catheter tube 360 can be correlated with an interior diameter of the lumen 358 and an inner wall 364 of the hollow cylindrical body 354, such that the temperature sensors 362 abut the inner wall 364 of the ablation tip 352. For instance, the catheter tube 360 can have the temperature sensors 362 disposed on the catheter tube 360 and the catheter tube 360 can be inserted into the lumen 358 of the ablation tip 352, placing the temperature sensors 362 in contact with the inner wall 364 of the ablation tip 352. Accordingly, as the ablation tip 352 is heated to perform an ablation, the temperature sensors 362 can measure a temperature of the ablation tip 352. In some embodiments, the temperature sensors 362 can be thermocouples and/or RTDs (e.g., thermistors), among other types of temperature sensors.

In some embodiments, as discussed herein, the temperature sensors 362 can be distributed across a surface of the catheter tube 360, such that a temperature across multiple areas of the ablation tip 352 can be determined. In some embodiments, a thermally insulating layer can be disposed between the surface of the catheter tube 360 and the temperature sensors 362 and/or the catheter tube 360 can be formed from a thermally insulating material. Accordingly, this can increase an accuracy at which the temperature sensors 362 collect temperature data from the ablation tip 352, because the main source of thermal activity measured by the temperature sensors 362 will come from the ablation tip 352 and not the catheter tube 360, which is thermally insulated from the temperature sensors 362.

As depicted, in some embodiments, the ablation tip can include irrigation ports 366-1, 366-2, 366-3. However, in some embodiments, no irrigation ports 366-1, 366-2, 366-3 can be included in the ablation tip 352. In some embodiments, based on a determination of a temperature of the ablation tip 352 made from a signal obtained from one of the RTDs 362, an energy supplied to the ablation tip 352 and/or an amount of irrigation fluid supplied via the irrigation ports 366-1, 366-2, 366-3 can be adjusted. As previously discussed in relation to Fig. 8, each RTD 362 can be electrically coupled to a processing unit via a pair of leads. For example, with reference to the RTD 362-1, the RTD 362-1 can be electrically coupled with a processing unit via a pair of leads 368-1, 368-2.

Fig. 10 is a side view of a temperature enabled ablation balloon 380, in accordance with embodiments of the present disclosure. The temperature enabled ablation balloon 380 can be disposed about a catheter shaft 384, which extends along an elongate axis defined by line cc, and can be inflated with a cryogenic fluid to perform a pulmonary vein isolation, in some embodiments. As depicted, a plurality of temperature sensors 386-1, 386-2, 386-3, 386-4, hereinafter referred to in the plural as temperature sensors 386, can be disposed on the ablation balloon 382. In some embodiments, the temperature sensors 386 can be thermocouples and/or RTDs, among other types of temperature sensors. In an example, the temperature sensors 386 can be printed on an exterior surface and/or an interior surface of the ablation balloon 382.

As depicted, the temperature sensors 386 can be concentrically disposed along the ablation balloon 382 along an outer most circumference of the ablation balloon 382. For example, the temperature sensors 386 can be uniformly disposed about a point 388 on the axis cc. For example, the temperature sensors 386 can be disposed on the ablation balloon 382 at a same axial distance along the axis cc. Alternatively, in some embodiments, the temperature sensors 384 can be disposed at varying axial distances along the axis cc. For example, some of the temperature sensors 394 can be disposed proximally or distally with respect to the point 388 on the axis cc.

Each one of the temperature sensors can be electrically coupled with a processing unit via a pair of leads. In an example, with reference to the temperature sensor 386-4, the temperature sensor 386-4 can be coupled with the processing unit via a first lead 390-1 and a second lead 390-2.

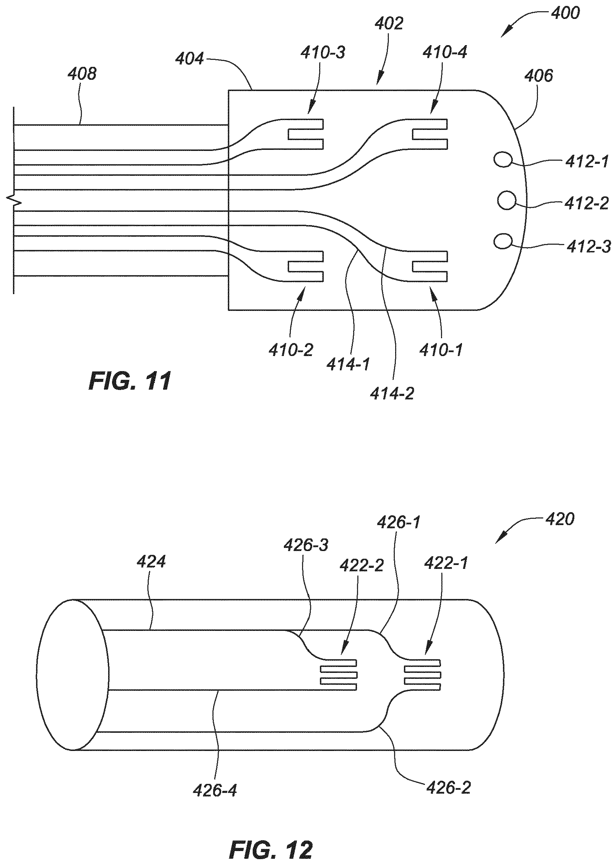

Fig. 11 is a diagrammatic isometric view of a temperature enabled catheter ablation tip 400 that includes temperature sensors disposed on an ablation tip 402, in accordance with embodiments of the present disclosure. The temperature enabled catheter ablation tip 400 can include an ablation tip 402. In some embodiments, the ablation tip 402 can be a radio frequency ablation tip. The ablation tip 402 can include a hollow cylindrical body 404 and a domed tip 406. The ablation tip 402 can be disposed at the end of a stem 408 in some embodiments. The ablation tip 402 can include a plurality of temperature sensors 410-1, 410-2, 410-3, 410-4, hereinafter referred to in the plural as temperature sensors 410, disposed in an array on the ablation tip 402. In some embodiments, the stem 408 can be connected to other components located more proximally with respect to the stem 408 and the catheter ablation tip 400. For example, the stem 408 can be connected to a catheter shaft, fluid lumen, etc. in some embodiments.

In some embodiments, the temperature sensors 410 can be printed on an exterior surface or an interior surface of the ablation tip 402, as discussed herein. In an example, where the temperature sensors 410 are printed on an interior surface of the ablation tip 402, the ablation tip 402 and specifically the hollow cylindrical body 404 and the domed tip 406 can define a lumen. For example, an interior surface of the hollow cylindrical body 404 and an interior surface of the domed tip 406 can define the lumen. The temperature sensors 410 can be placed on the interior surface, such that the temperature sensors 410 are in contact with the ablation tip 402. In some embodiments, the temperature sensors 410 can be placed on the exterior surface of the ablation tip 402. Accordingly, as the ablation tip 402 is heated to perform an ablation, the temperature sensors 410 can measure a temperature of the ablation tip 402. In some embodiments, as discussed herein, the temperature sensors 410 can be distributed across an interior or exterior surface of the ablation tip 402, such that a temperature across multiple areas of the ablation tip 402 can be determined.

As depicted, in some embodiments, the ablation tip can include irrigation ports 412-1, 412-2, 412-3. However, in some embodiments, no irrigation ports 412-1, 412-2, 412-3 can be included in the ablation tip 402. In some embodiments, based on a determination of a temperature of the ablation tip 402 made from a signal obtained from one of the temperature sensors 410, an energy supplied to the ablation tip 402 and/or an amount of irrigation fluid supplied via the irrigation ports 412-1, 412-2, 412-3 can be adjusted. As previously discussed in relation to Fig. 8, each temperature sensor 410 can be electrically coupled to a processing unit via a pair of leads. For example, with reference to the temperature sensor 410-1, the temperature sensor 410-1 can be electrically coupled with a processing unit via a pair of leads 414-1, 414-2. As previously discussed herein, the temperature sensors 410 can be, for example, thermocouples and/or RTDs.

Fig. 12 is a diagrammatic isometric view of a temperature enabled catheter tube 420 with temperature sensors 422-1, 422-2 that share a common lead 424 (e.g., ground), in accordance with embodiments of the present disclosure. The temperature enabled catheter tube 420 can include features that are similar to or the same as those discussed herein, for example, in relation to Fig. 8. As depicted, the temperature sensors 422-1, 422-2 can be RTDs, however, the temperature sensors 422-1, 422-2 can be other types of temperature sensors in some embodiments. The temperature sensors 422-1, 422-2 can share a common lead 424 in some embodiments. In an example, each temperature sensor 422-1, 422-2 can be connected to a processing unit via a pair of leads. For instance, the first temperature sensor 422-1 can be connected to the processing unit via a first lead 426-1 and a second lead 426-2 and the second temperature sensor 422-2 can be connected to the processing unit via a third lead 426-3 and a fourth lead 426-4. In some embodiments, the first lead 426-1 and the third lead 426-3 can be electrically coupled with the common return 424.

Some embodiments of the present disclosure can include a multiplexing chip, which can be used to cycle through the first temperature sensor 422-1 and the second temperature sensor 422-2. For example, the first temperature sensor 422-1 and/or the second temperature sensor 422-2 can be activated via the multiplexing chip, allowing for a temperature to be determined via the first temperature sensor 422-1 and/or the second temperature sensor 422-2.

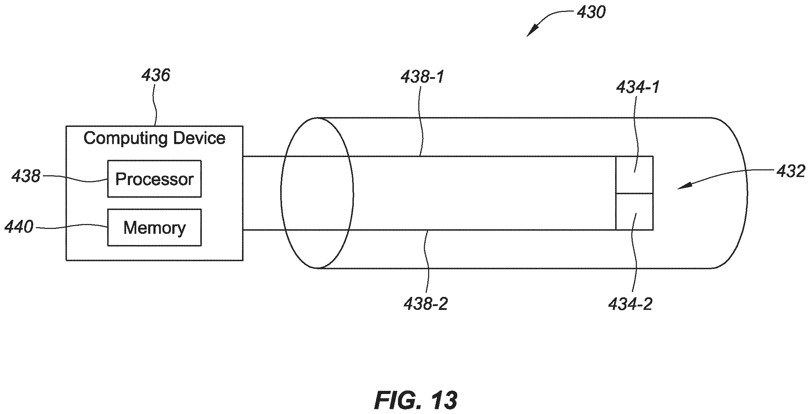

Fig. 13 is a diagrammatic isometric view of a temperature enabled catheter tube 430 with a temperature sensor 432 in communication with a computing device 436, in accordance with embodiments of the present disclosure. As depicted, the temperature sensor 432 can be disposed on the catheter tube 430 and can be a thermocouple 432. However, the temperature sensor 332 can be an RTD (e.g., thermistor) or another type of temperature sensor. The thermocouple 432 can include a first thermocouple element 434-1 and a second thermocouple element 434-2. The first thermocouple element 434-1 and the second thermocouple element 434-2 can be formed from dissimilar metals and one of the metals can be a type of metal that produces a temperature dependent response. The first thermocouple element 434-1 can be connected to the computing device 436 via a first lead 438-1 and the second thermocouple element 434-2 can be connected to the computing device 436 via a second lead 438-2. In some embodiments, the computing device 436 can analyze the signals received from the thermocouple 432 and can determine a temperature based on the analyses of the received signal.

In some embodiments, the computing device 436 can include a processor 438 and memory 440. The memory 440 can be a non-transitory computer readable medium that stores instructions executable by the processor 438 to perform a particular function. For example, the memory 440 can store instructions that are executable by the processor 438 to analyze the signals received from the thermocouple 432.

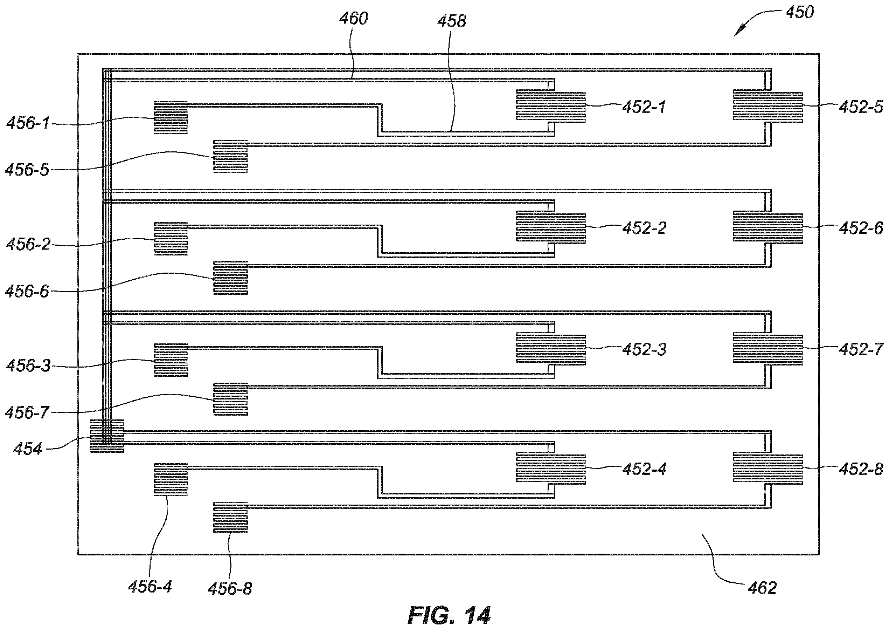

Fig. 14 is a top view of an array 450 of temperature sensors 452-1, 452-2,..., 452-8 that share a common return lead 454, in accordance with embodiments of the present disclosure. In some embodiments, the temperature sensors 452-1, 452-2,..., 452-8 can be axially aligned, as depicted, and/or can be axially offset, as discussed in relation to Fig. 8b. The temperatures sensors 452-1, 452-2,..., 452-8 can be RTDs, in some embodiments. In some embodiments, the temperature sensors 452-1, 452-2,..., 452-8 can be thermocouples and/or thermistors. As depicted, a first row of temperature sensors 452-1, 452-2, 452-3, 452-4 is laterally aligned with one another and a second row of temperature sensors 452-5, 452-6, 452-7, 452-8 is laterally aligned with one another, although in some embodiments one or more of the temperature sensors 452-1, 452-2,..., 452-8 can be laterally offset with respect to one another. Each one of the temperature sensors 452-1, 452-2,..., 452-8 can include a source lead 456-1, 456-2,..., 456-8. In some embodiments, a terminal end of the source lead 456-1, 456-2,..., 456-8 can include a source pad, as depicted, which can allow for additional leads to be coupled to a respective one of the source leads 456-1, 456-2,..., 456-8. For instance, additional leads can be coupled to respective ones of the source leads 456-1, 456-2,..., 456-8 and can be extend proximally along a length of a catheter shaft and can be connected to a power source to provide power to one or more of the temperature sensors 452-1, 452-2,..., 452-8. With particular reference to the temperature sensor 452-1, a source lead 456-1 (e.g., source contact pad) can include a distally extending source lead 458, which can be electrically coupled with the source lead 456-1 and the temperature sensor 452-1 and can provide power to the temperature sensor 452-1. A proximally extending return lead 460 can extend from the temperature sensor 452-1 and can be electrically coupled with the common return lead 454 (e.g., common return contact pad). In some embodiments, a measurement of resistance can be made based on an input signal provided to each one of the temperature sensors 452-1, 452-2,..., 452-8 and a return signal provided via the common return lead 454. Based on the measurement of resistance, a temperature at each one of the temperature sensors 452-1, 452-2,..., 452-8 can be determined.

In some embodiments, the array 450 of temperature sensors 452-1, 452-2,..., 452-8 can be disposed on a substrate 462. In some embodiments, the substrate 462 can be a surface associated with a catheter ablation tip. For example, the substrate 462 can be an interior surface of an ablation tip and/or an exterior surface of the ablation tip. In some embodiments, the substrate 462 can be a planar sheet of material (e.g., film) on which the temperature sensors 452-1, 452-2,..., 452-8 and associated leads are formed. In some embodiments, where the substrate 462 is a planar sheet of material on which the temperature sensors 452-1, 452-2,..., 452-8 and associated leads are formed, the substrate can be adhered to an interior or exterior surface of the catheter ablation tip.

In some embodiments, where the array 450 of temperature sensors 452-1, 452-2,..., 452-8 is disposed on a catheter ablation tip, the common return lead 454 can be electrically coupled with the catheter ablation tip. In an example, the catheter ablation tip can include one or more tip leads that extend proximally along the catheter shaft. Accordingly, the one or more tip leads can act as return leads that are electrically coupled to the catheter ablation tip and thus to the common return lead 454. Additionally, in some embodiments, a radiofrequency (RF) signal that is used to drive the ablation tip can provide power to the temperature sensors 452-1, 452-2,..., 452-8. Accordingly, power can be provided to each one of the temperature sensors 452-1, 452-2,..., 452-8 via the RF signal and each one of the temperature sensors 452-1, 452-2,..., 452-8 can include a proximally extending return lead (e.g., proximally extending return lead 460) that in some embodiments can be electrically coupled to the catheter ablation tip. In such an embodiment, the array may or may not utilize the source leads 456-1, 456-2,..., 456-8 to provide power to each one of the temperature sensors 452-1, 452-2,..., 452-8.

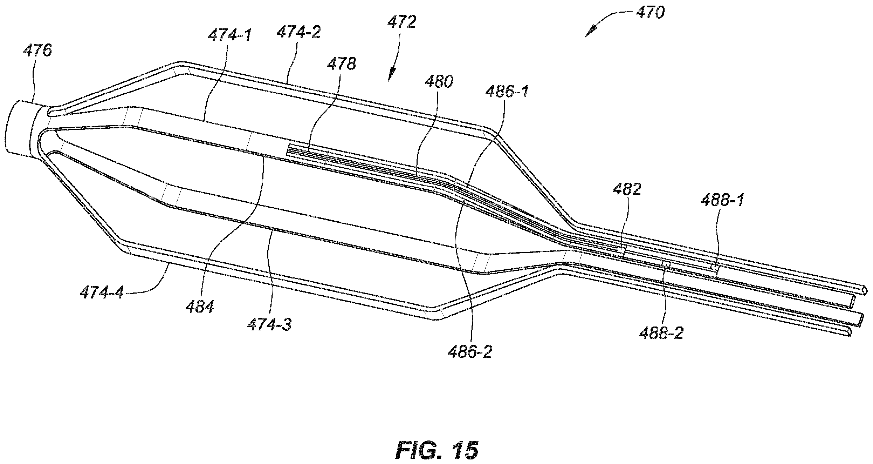

Fig. 15 is a side view of a radial ablation tip 472, according to various embodiments of the present disclosure. The radial ablation tip 472 can be connected to a distal end of a catheter shaft (not depicted). The radial ablation tip 472 can be similar to that included in the EnligHTN™ Multi-Electrode Renal Denervation System, produced by St. Jude Medical, further discussed in U.S. Application no. 14/258,407, which is incorporated by reference as though full set forth herein. The radial ablation tip 472 can include a plurality of radially expanding members 474-1, 474-2, 474-3, 474-4 that extend distally and axially from a connector (not depicted), which is attached to the distal end of the shaft. In some embodiments, a distal end of each of the radially expanding members 474-1, 474-2, 474-3, 474-4 can be connected to a connector 476. In some embodiments, the shaft can be extended or retracted in relation to the connector 476, causing the radially expanding members 474-1, 474-2, 474-3, 474-4 to radially expand or radially retract.

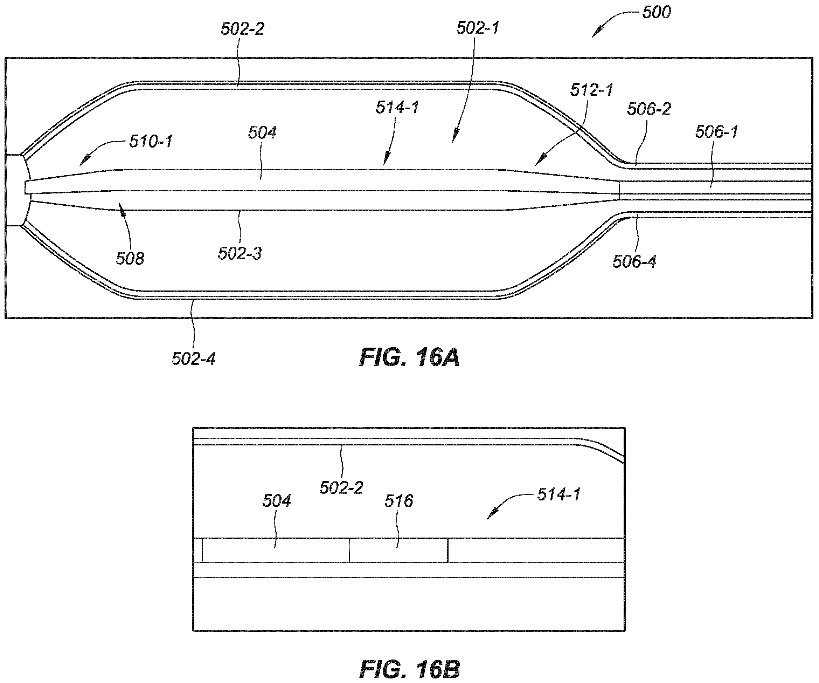

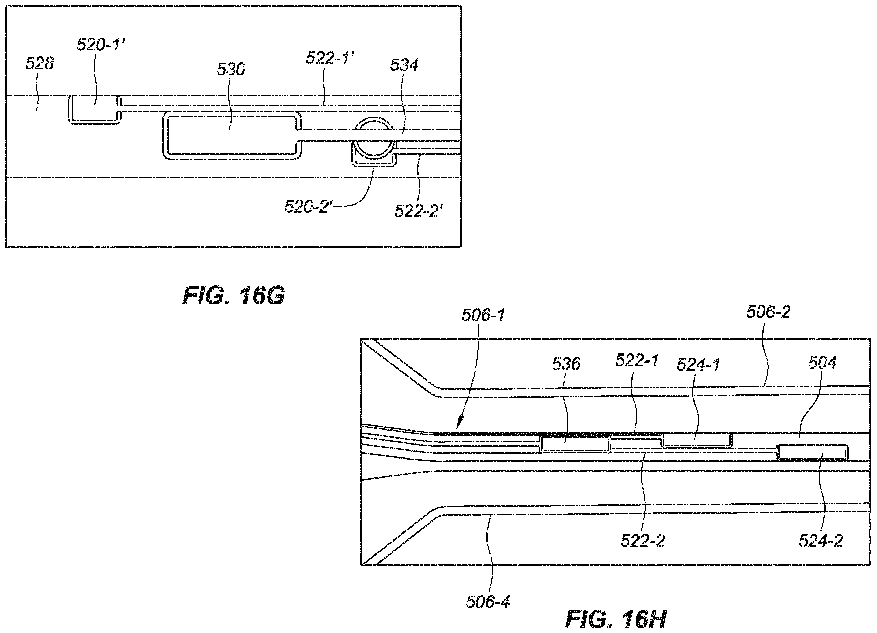

In some embodiments, each one of the radially expanding members 474-1, 474-2, 474-3, 474-4 can include one or more electrodes 478 disposed thereon. Only electrode 478 is depicted for ease of clarity. As the radially expanding members 474-1, 474-2, 474-3, 474-4 expand, the electrode(s) 478 can contact a lumen in which the radial ablation tip 472 is disposed. For example, the radial ablation tip 472 can be disposed in a renal artery and the radial ablation tip 472 can be expanded to cause the electrode(s) 478 to contact an inner wall of the renal artery. Upon contact with the inner wall, the electrode(s) 478 can perform a sensing and/or therapeutic function. For example, the electrode(s) 478 can sense electrical signals passing along the renal artery and/or can perform an ablation to the renal artery. In some embodiments, the electrode(s) 478 can be three-dimensional electrodes, such as those discussed in relation to Figs. 4A to 5B. For example, the electrode(s)s 478 can extend upwards from a surface of each one of the radially expanding members 474-1, 474-2, 474-3, 474-4. The electrode(s) 478 can be formed as discussed in relation to Figs. 2 to 5B and/or in relation to Figs. 16A to 16J.

In some embodiments, the electrode(s) 478 can include one or more electrical leads 480 that connect the electrode 478 to a contact pad 482, which can be disposed on a proximal end of one of the radially expanding members 474-1, 474-2, 474-3, 474-4 (e.g., radially expanding member 474-1). As depicted the one or more electrical leads 480 can extend from the electrode 478 proximally down the radially expanding member 474-1. The electrical leads and their construction is further discussed herein, in relation to Figs. 16A to 16J.

In some embodiments, the electrode(s) 478 can include a temperature sensor 484 such as that depicted and discussed in relation to Figs. 2 to 3B. In an example, the temperature sensor 484 can be disposed underneath one or more of the electrode(s) 478 and/or formed within one or more of the electrode(s) 478. The temperature sensor 484 can thereby measure a temperature of one or more of the electrode(s) 478. In some embodiments, the temperature sensor 484 can include one or more electrical leads 486-1, 486-2 that connect the temperature sensor 484 to one or more respective contact pads 488-1, 488-2, which can be disposed on a proximal end of one of the radially expanding members 474-1, 474-2, 474-3, 474-4 (e.g., radially expanding member 474-1). As depicted the electrical leads 486-1, 486-2 can extend from the temperature sensor 484 proximally down the radially expanding member 474-1. The electrical leads and their construction is further discussed herein, in relation to Figs. 16A to 16J.

Fig. 16A depicts a side view of an exemplary radial ablation tip 500 that includes a dielectric coating 504, according to various embodiments of the present disclosure. The radial ablation tip 472 can include an understructure formed from a plurality of radially expanding members 502-1, 502-2, 502-3, 502-4 that extend distally and axially from a set of connective members 506-1, 506-2, 506-3 (not depicted), 506-4. In some embodiments, the understructure can be formed from a flexible or spring-like material such as Nitinol and/or a flexible substrate, as discussed herein. In some embodiments, examples of the dielectric material 504 can include a polyimide, such as a polyimide (e.g., PI-2771 or HD-4004 available from HD Microsystems) and/or an epoxy (e.g., SU8 epoxy available from MicroChem Corp), etc. can be used in accordance with design and end-use requirements. Other polyimide materials can include parylene. In some embodiments where the understructure is formed from an electrically conductive material, the dielectric material 504 can electrically insulate the conductive traces, as discussed herein, from the electrically conductive material. In some embodiments, an aerosol jet can be used to deposit the dielectric material 504 on a surface of each one of the radially expanding members 502-1, 502-2, 502-3, 502-4 and connective members 506-1, 504-2, 506-3 (not depicted), 506-4. However, the dielectric material 504 can be deposited in other ways, as well.

In some embodiments, the dielectric material 504 may not coat an entirety of the understructure. For example, the dielectric material 504 can be disposed along each radially expanding member up to a distal transition point 508 of each one of the radially expanding members 502-1, 502-2, 502-3, 502-4. With respect to the radially expanding member 502-1, the dielectric material 504 can coat the connective member portion 506-1 of the radially expanding member 502-1, a proximal transition portion 512-1 of the radially expanding member 502-1, and a central portion 514-1 of the radially expanding member 502-1. The dielectric coating 504 can stop at a transition between the distal transition portion 510-1 and the central portion 514-1. In an example, further steps can include depositing a temperature sensor and/or an electrode along the dielectric material 504. In some embodiments, where a temperature sensor and/or electrode are deposited on distal transition portion 510-1, the dielectric material 504 can be extended further distally along each one of the radially expanding members 502-1, 502-2, 502-3, 502-4, for example, such that it covers the distal transition portion 510-1, thus providing for an insulating material between the temperature sensor and/or electrode and the understructure forming the radially expanding members 502-1, 502-2, 502-3, 502-4.

Fig. 16B depicts a temperature sensor disposed on the central portion 514-1 of the radially expanding member 502-1, on top of the dielectric material 504, in accordance with embodiments of the present disclosure. In an example, the temperature sensor 516 can be a thermistor, in some embodiments. The temperature sensor 516 can be formed through the deposition of a conductive material onto the dielectric material. In some embodiments, the conductive material can be a platinum material. In some embodiments, the temperature sensor 516 may only cover a portion of the central portion 514-1 of the radially expanding member 502-1. For example, in some embodiments, portions of the central portion 514-1 or other portions of the radially expanding member 501-2 can be masked, to prevent the deposition of the conductive material (e.g., platinum) on the portions of the radially expanding member 501-2 for which it is not desired to have the conductive material deposited.

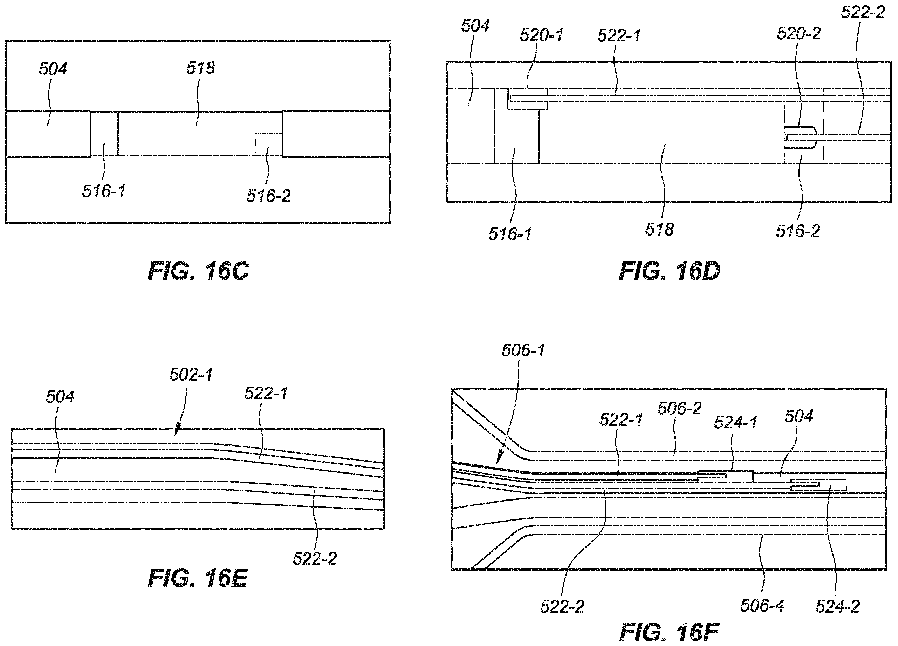

Fig. 16C depicts a second dielectric layer 518 deposited over a portion of the temperature sensor 516, in accordance with embodiments of the present disclosure. In some embodiments, a portion of the temperature sensor 516 can be coated with a second dielectric layer 518, leaving one or more temperature sensor contact pads 516-1, 516-2 exposed. In some embodiments, a first temperature sensor contact pad 516-1 can be located at a distal end of the temperature sensor 516 and a second temperature sensor contact pad 516-2 can be located at a proximal end of the temperature sensor 516. As depicted in Fig. 16D, a first and second electrical contact 520-1, 520-2 can be electrically coupled with the first and second temperature sensor contact pads 516-1, 516-2. The first and second electrical contacts 520-1, 520-2 can connect first and second conductors 522-1, 522-2, respectively, to the temperature sensor 516. The first and second conductors can extend proximally from the temperature sensor 516, along each one of the plurality of radially expanding members (e.g., radially expanding member 502-1), as depicted in Figs 16D, 16E, and 16F. As further depicted in Fig. 16F, the proximal end of each one of the first and second conductors 522-1, 522-2 can terminate at a respective one of a first and second electrical contact terminal 524-1, 524-2. In some embodiments, another set of electrical leads can be connected to the first and second electrical contact terminals 524-1, 524-2 and to a computing device, which can process a signal received from the temperature sensor 516. For example, the computing device can determine a temperature from the signal received from the temperature sensor 516.

Fig. 16G depicts a dielectric coating 528 disposed over the temperature sensor 516 depicted in Fig. 16B, with an electrode 530 disposed over the dielectric coating 528, in accordance with embodiments of the present disclosure. In some embodiments, the electrode 530 can be formed from a conductive material. In an example, the electrode 530 can be formed from platinum. The electrode 530 can be deposited on top of the dielectric coating 528, which insulates the electrode 530 from the temperature sensor 516, and its first and second electrical conductors 522-1', 522-2'. In some embodiments, an electrode lead 534 can be electrically coupled with the electrode 530 and can extend proximally along the radially expanding member 502-1, towards a proximal end of the radial ablation tip 500 and can terminate on the connective member 506-1 at an electrical contact terminal 536. In some embodiments, the electrode 530 can be electrically coupled to a medical device control system, as depicted in Fig. 1. In some embodiments, the electrode 530 can be an ablation electrode and/or a diagnostic electrode, which can sense electrical rhythms of the heart and/or a tissue.

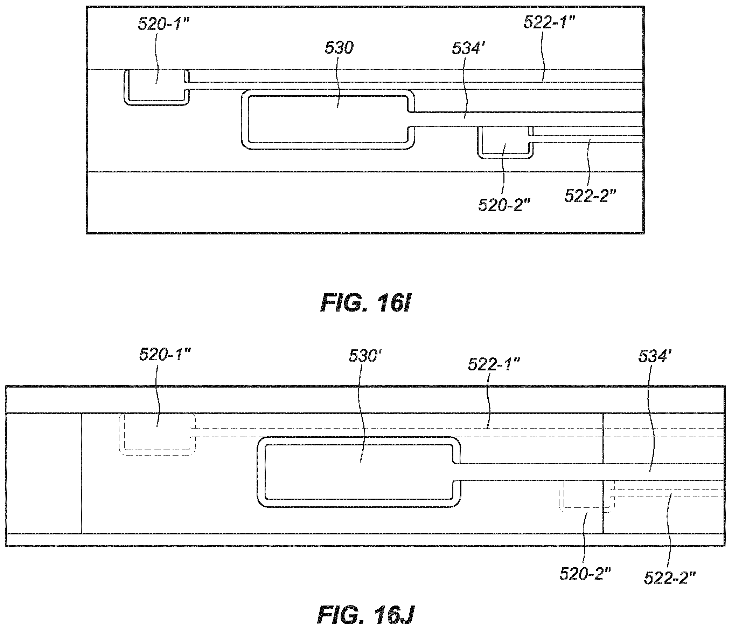

Fig. 16I depicts a dielectric coating disposed over the areas surrounding the electrode 530, in accordance with embodiments of the present disclosure. In some embodiments, a dielectric material can be disposed over portions surrounding the electrode 530. For example, a dielectric material 540 can be disposed over the electrode lead 534' and/or conductors 522-1", 522-2" and/or electrical contacts 520-1", 520-2". However, the electrode 530 itself may remain uncoated with the dielectric material. As depicted in Fig. 16J, the electrode 530' has been coated with a highly conductive material. For example, the electrode 530' can be coated with gold, in some embodiments. In some embodiments, the conductive layering might be other materials in lieu of or in addition to gold. Depending on the substrate, sometimes other metal materials may be needed for interstitial adhesion layers between the substrate and the conductive layer. Chrome and/or Nickel can be used in some embodiments. This range would be for the whole conductor layer. In some embodiments, the substrate can be a polymer or metal, dielectric, a conductor (e.g., cu), dielectric (e.g., with exposed electrodes), and then the electrode (Cu) and Gold layer at the top (for example). As further depicted in Fig. 16J, and as further discussed herein, the coated electrode 530' can have a three dimensional profile, which extends above a surface of a respective radially expanding member on which the coated electrode is disposed.

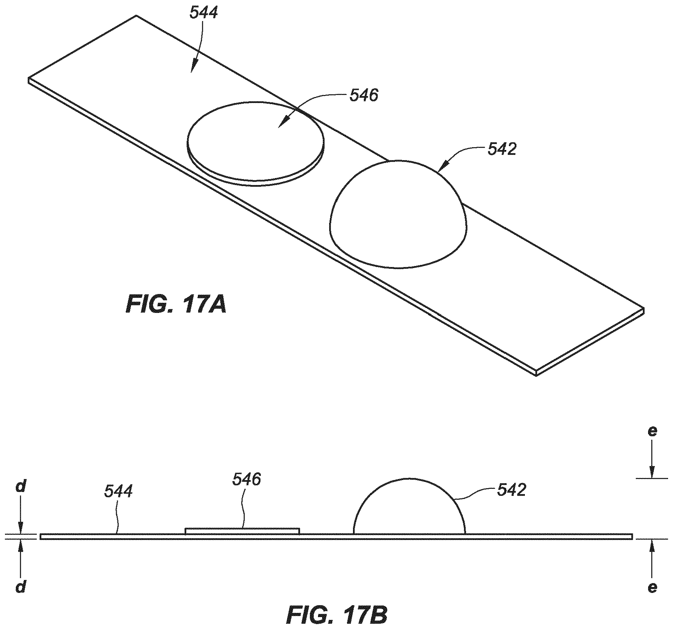

Fig. 17A depicts an elevated electrode 542 disposed on a substrate 544, in accordance with embodiments of the present disclosure, next to a traditional electrode 546 disposed on the substrate 544. In some embodiments, the substrate 544 can be a flexible circuit, however, the substrate 544 can be formed from other materials, such as a metal. In some embodiments, systems, such as the EnSite™ NavX™ navigation system, produced by St. Jude Medical, Inc., can utilize electrodes that have low impedance. As the impedance of the electrode increases, the noise on the electrograms produced by the system can become worse to the point that the electrograms can be unusable. Additionally, as the impedance of the electrode decreases, the accuracy of the EnSite™ NavX™ navigation system decreases, when located with electrodes of low impedance. Balancing of the reduction of noise in electrograms with maintaining of the location accuracy of the EnSite™ NavX™ navigation system can be competing priorities when measuring local electrograms.

Large electrodes (which have low impedance) can cover comparatively large areas of the myocardium and/or other tissue and can blur an electrogram map, as a result from electrical signals being collected from a larger area of the tissue surface. This can make diagnosis of a particular condition difficult or inaccurate, because the electrode may not be able to pinpoint an area producing a particular electrical signal. For example, the electrode may be disposed over a larger area, which can cause the electrode to collect electrical signals not from one particular area, but over the larger area. However, smaller electrodes can increase an impedance associated with the electrode. While prior attempts have been made to add coatings or surface area modifications to increase the overall surface area without changing the electrode size, this requires additional processing steps and/or harsh chemicals.

To overcome the electrode size versus impedance issue, some embodiments can include an electrode that can protrude from a surface of a substrate associated with a medical device. In some embodiments, the substrate can be disposed on a distal portion of a catheter. In some embodiments, the substrate can be disposed on an end effector located at a distal portion of the catheter. In some embodiments, the electrode can be include a spherical cap and/or can be configured as a cylindrical shape, versus a planar circle and/or oval, which can lower the overall impedance, help ensure better tissue contact, and can be accomplished with minimal extra process steps and/or harsh chemicals.

As depicted in Figs. 17A and 17B, the traditional electrode 544 can be a planar surface that extends a negligible height above a surface of the substrate 546, represented by the line dd. In an example, the traditional electrode 546 can extend a height in a range from 1 to 50 microns and in some cases 12 to 154 microns above the surface of the substrate 544 and can have a planar face that extends parallel with the surface of the substrate 544, in some embodiments. In contrast, the elevated electrode 542 can extend a greater height above the surface of the substrate 544, represented by the line ee. In some embodiments, the elevated electrode 542 can extend a height above the surface of the substrate 544 in a range from 12 to 625 microns. In some embodiments, at a height of 625 microns, additional process steps may be required. For example, a mechanical bumpout can be formed on a substrate 544 and the elevated electrode 542 can be formed on top of the mechanical bumpout to provide extra height. In some embodiments, the elevated electrode 542 can extend a height above the surface of the substrate 544 in a range from 50 to 250 microns. In some embodiments, the elevated electrode 542 can extend a height above the surface of the substrate 544 in a range from 50 to 70 microns. All individual subranges are included in embodiments of the present disclosure.

The elevated electrode 542 can be configured as a spherical cap, in some embodiments, as depicted in Figs. 17A and 17B. In an example, the elevated electrode 542 can be a hemisphere and/or a partial hemisphere that is attached to the substrate. In some embodiments, a planar portion of the hemisphere can be attached to the substrate, such that the curved hemispherical portion of the elevated electrode 542 extends above the substrate 544.

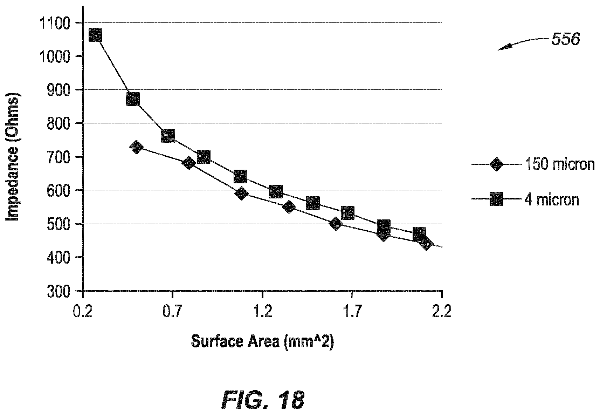

In some embodiments, the elevated electrode 542 can be formed from a conductive material. For example, in some embodiments, the elevated electrode 542 can be formed from gold. However, solely constructing the elevated electrode 542 from gold can add additional cost to construction and can provide for a more complicated process associated with construction of the elevated electrode. Further, a total height of the electrode can be limited when the elevated electrode 542 is constructed entirely from a material, such as gold. In some embodiments, as depicted in Fig. 18D, the elevated electrode 542 can be constructed with a conductive core 548. In some embodiments, the conductive core 548 can be formed from a first conductive material. In some embodiments, the conductive core 548 can be formed from copper. In an example, the conductive core 548 can also be a spherical cap, which can be a hemisphere and/or a partial hemisphere. In some embodiments, a thickness of the conductive core 548 can be in a range from 10 microns to 150 microns, although embodiments are not so limited and the thickness can be lesser or greater than the provided range. In some embodiments, a thickness of the conductive core 548 can be in a range from 10 microns to 575 microns, with a combination of a thickness of the conductive core 548 and a mechanical bumpout being 575 microns.

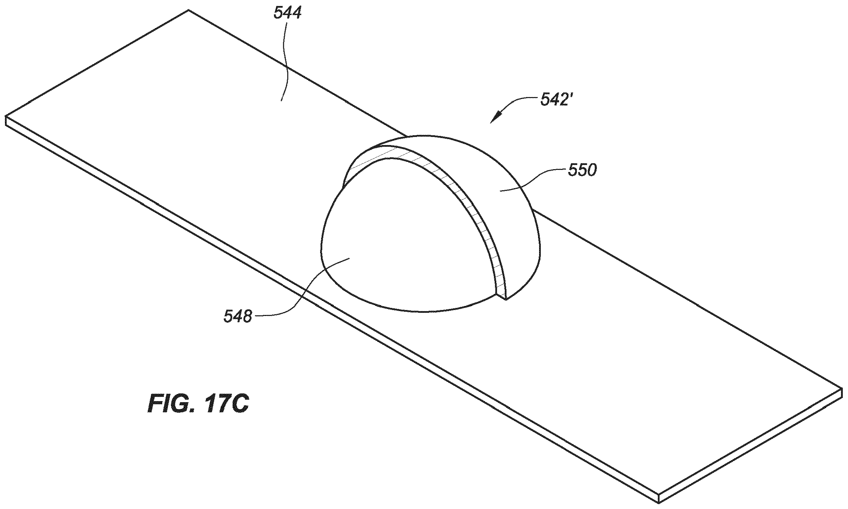

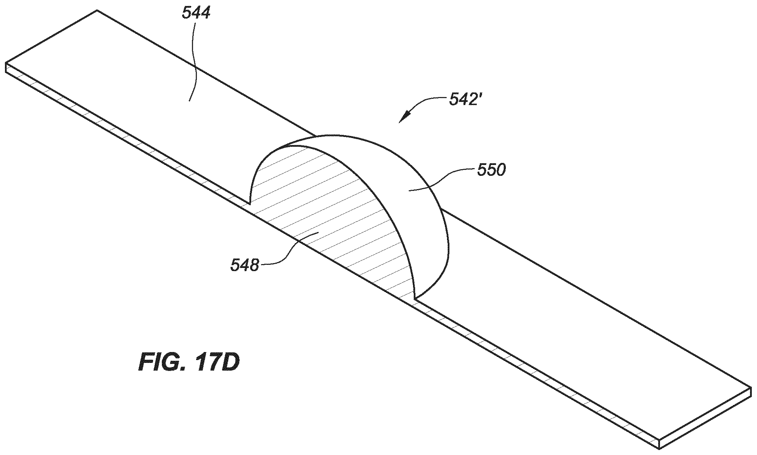

As further depicted in Figs. 17C and 17D, a conductive coating 550 can be disposed over the conductive core 548, which can uniformly coat an exterior of the conductive core 548, in some embodiments. The conductive coating 550 can be formed from a second conductive material, which in some embodiments can have a greater conductivity than the first conductive material that forms the conductive core 548. The conductive coating 550 can be formed from gold, in some embodiments, which can provide for a decreased impedance associated with the elevated electrode 542', while still providing for an electrode with a size that can provide for targeted measurements of electrical signals associated with a tissue. In some embodiments, the conductive coating 550 can have a thickness in a range from 2 microns to 4 microns, although embodiments are not so limited and the thickness can be lesser or greater than the provided range. In some embodiments, the conductive coating 550 can have a thickness in a range from 1 microns to 50 microns.

Fig. 18 depicts a graphical chart 556 representing a difference in impedance of a traditional type electrode with a height of 4 microns versus that of an elevated electrode with a height of 150 microns, in accordance with embodiments of the present disclosure. As depicted, the graphical chart indicates that an elevated electrode with a height of 150 microns, in accordance with embodiments of the present disclosure, can provide for an impedance that is lower than a traditional type of electrode with a height of 4 microns. As further depicted, the 150 micron elevated electrode can have a same surface area as the 4 micron electrode, while having a lower impedance. This is proven to be especially true for electrodes with a smaller surface area, as depicted by the graphical chart 556. Thus, embodiments of the present disclosure can provide for an elevated electrode that can collect electrical signals from a particular area, while further providing for an impedance level associated with the electrode that is low, resulting in accurate and usable electrograms.

One or more embodiments of the present disclosure can be combined. For example, embodiments of the present disclosure can provide an electrode and temperature sensor that is incorporated into one element. In some embodiments, the electrode and temperature sensor that is incorporated into one element can include a three-dimensional profile, as discussed herein. In some embodiments, the electrode and temperature sensor can be arranged in an array with each electrode and/or temperature sensor being connected to a processing unit via printed leads. Embodiments are described herein of various apparatuses, systems, and/or methods. Numerous specific details are set forth to provide a thorough understanding of the overall structure, function, manufacture, and use of the embodiments as described in the specification and depicted in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment(s) is included in at least one embodiment. Thus, appearances of the phrases "in various embodiments," "in some embodiments," "in one embodiment," or "in an embodiment," or the like, in places throughout the specification, are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

Although various embodiments have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and can include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting. Changes in detail or structure can be made without departing from the spirit of the disclosure as defined in the appended claims.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

**Claims**

1. A medical device, comprising:

    an elongate shaft extending along a shaft longitudinal axis and comprising a shaft proximal portion and a shaft distal portion;
    an electrode (100) disposed on the shaft distal portion;
    a first conductor lead (104-1) and a second conductor lead (104-2), each of the conductor leads electrically coupled to the electrode;
    a thermocouple junction (102) formed via a thermocouple conductor (106) electrically coupled to the electrode (100) and the first conductor lead (104-1).

2. The medical device of claim 1, wherein the first conductor lead (104-1), the second conductor lead (104-2), and the thermocouple conductor (106) are disposed between the shaft distal portion and the electrode.

3. The medical device of claim 1, wherein the first conductor lead (104-1), the second conductor lead (104-2), and the thermocouple conductor (106) are disposed within the electrode.

4. The medical device of claim 1, wherein the first conductor lead (104-1) comprises a first conductor tip (108-1) and the thermocouple conductor (106) comprises a thermocouple tip (110).

5. The medical device of claim 4, wherein the thermocouple junction comprises an electrical junction between the first conductor tip (108-1) and the thermocouple tip (110).

6. The medical device of claim 5, wherein:

    the second conductor lead (104-2) comprises a second conductor tip (108-2); and
    the second conductor tip is disposed adjacent to the trace temperature sensor.

7. The medical device of claim 1, wherein the electrode is disposed on a substrate that includes a three-dimensional profile portion.

8. The medical device of claim 7, further comprising a plurality of electrodes, each of the electrodes including respective conductor leads (104-1, 104-2) and thermocouple conductors (106) that form respective thermocouple junctions on each one of the plurality of electrodes, wherein the plurality of electrodes are formed in an array disposed on the shaft distal portion.

9. The medical device of any preceding claim, further comprising:

    a balloon disposed along the shaft distal portion; and
    a plurality of temperature sensors disposed on the balloon.

10. The medical device of claim 9, wherein the plurality of temperature sensors are resistance temperature detectors.

11. The medical device of claim 9 or 10, wherein the temperature sensors are disposed on at least one of an interior surface and an exterior surface of the balloon.

12. The medical device of claim 9 or 10, wherein the temperature sensors are concentrically disposed along the balloon about an outer most circumference of the ablation balloon.

FIG. 1

**FIG. 2**

**FIG. 3A**

**FIG. 3B**

220-a

222-a

224-a

226-a

**FIG. 4A**

220-b

226-b

228-b

224-b

232-2

230-b

222-b

234-b

232-1

**FIG. 4B**

220-c

226-c

228-c

232-2

222-c

236

230-c

234-c

232-1

**FIG. 4C**

**FIG. 5A**

**FIG. 5B**

**FIG. 6**

**FIG. 7A**

**FIG. 7B**

FIG. 8A

FIG. 8B

**FIG. 8C**

**FIG. 8D**

**FIG. 9**

**FIG. 10**

**FIG. 11**

**FIG. 12**

FIG. 13

**FIG. 14**

EP 4 548 871 A2

EP 4 548 871 A2

FIG. 15

35

**FIG. 16A**

**FIG. 16B**

EP 4 548 871 A2

**FIG. 16C**

**FIG. 16D**

**FIG. 16E**

**FIG. 16F**

EP 4 548 871 A2

FIG. 16G

FIG. 16H

FIG. 16I

FIG. 16J

544

546

542

**FIG. 17A**

d

546

544

542

e

d

e

**FIG. 17B**

**FIG. 17C**

EP 4 548 871 A2

**FIG. 17D**

EP 4 548 871 A2

**FIG. 18**

EP 4 548 871 A2

**REFERENCES CITED IN THE DESCRIPTION**

**Patent documents cited in the description**

- US 331562
- US 088052
- US 911474
- US 258407

**Non-patent literature cited in the description**

- Printed Wearable Temperature Sensors for Health Monitoring. **HONDA**. Sensor. IEEE, 2014